# EUROPEAN PATENT SPECIFICATION

(11) **EP 3 426 771 B1**
(45) Date of publication and mention of the grant of the patent: **07.05.2025**
(21) Application number: 17709457.0
(22) Date of filing: 13.03.2017
(51) Int. Cl.: A61P 1/04, A61P 1/16, A61P 3/10, A61P 7/06, A61P 9/10, A61P 9/14, A61P 11/02, A61P 11/06, A61P 17/00, A61P 19/02, A61P 25/00, A61P 29/00, A61P 37/02, A61P 37/06, A61P 37/08, C12N 5/0786

(54) **NEW IMMUNOREGULATORY CELLS AND METHODS FOR THEIR PRODUCTION**
IMMUNREGULIERENDE ZELLEN UND VERFAHREN ZU DEREN HERSTELLUNG
CELLULES IMMUNORÉGULATRICES ET LEURS PROCÉDÉS DE PRODUCTION

(30) Priority: 11.03.2016 EP 16159985
(43) Date of publication of application: 16.01.2019
(60) Divisional application: 24202637.5
(73) Proprietor: Ferring Ventures SA, 1162 St. Prex (CH)
(72) Inventor: HUTCHINSON, James, 93051 Regensburg (DE); GEISSLER, Edward, 93173 Wenzenbach (DE)
(74) Representative: Eisenführ Speiser
(86) International application number: PCT/EP2017/055839
(87) International publication number: WO 2017/153607

(56) References cited:
- WO-A2-2015/048641
- ANCUTA PETRONELA ET AL: "Transcriptional profiling reveals developmental relationship and distinct biological functions of CD16+ and CD16- monocyte subsets", BMC GENOMICS, BIOMED CENTRAL LTD, LONDON, UK, vol. 10, no. 1, 27 August 2009 (2009-08-27), pages 403, XP021056212, ISSN: 1471-2164, DOI: 10.1186/1471-2164-10-403
- HUTCHINSON JAMES A ET AL: "Human regulatory macrophages", METHODS IN MOLECULAR BIOLOGY (CLIFTON, N.J.)UNITED STATES2010,, vol. 677, 1 January 2011 (2011-01-01), pages 181 - 192, XP009162998, ISSN: 1940-6029, DOI: 10.1007/978-1-60761-869-0_13
- HUTCHINSON JAMES A ET AL: "Human regulatory macrophages as a cell-based medicinal product", CURRENT OPINION IN ORGAN TRANSPLANTATION,, vol. 17, no. 1, 1 February 2012 (2012-02-01), pages 48 - 54, XP009162999, ISSN: 1531-7013

## Description

### FIELD OF THE INVENTION

The present invention relates to novel immunoregulatory macrophage cells which are useful in the treatment of different immunological and non-immunological diseases and conditions. The cells are characterized by a specific marker and activity pattern which distinguishes them from other cells. The invention also provides a process for preparing the immunoregulatory macrophage cells from blood monocytes. In a still further aspect, the invention relates to a pharmaceutical composition comprising the immunoregulatory macrophage cells of the invention.

### TECHNICAL BACKGROUND

Transferring immunoregulatory cells from a tolerant donor to non-tolerant recipient as a means of establishing tolerance in the recipient is a well-known technique in experimental immunology, but its clinical application is only now receiving serious attention [1]. At present, several immunoregulatory cell types are reaching the point of preclinical development, which will allow them to be investigated as immunosuppressive agents in early-phase clinical trials, including regulatory T cells [2], tolerogenic dendritic cells [3] and regulatory macrophages [4].

A broad spectrum of immunologic conditions may be amenable to treatment with cell-based immunoregulatory therapies, including T cell- and B cell-mediated autoimmune disease, chronic inflammatory disorders, graft-versus-host disease (GVHD), and transplant rejection. In these conditions, cell-based immunoregulatory therapies might reduce or even obviate the need for general immunosuppressive or anti-inflammatory therapy, thereby sparing patients its attendant complications. Because the kind of immunologic tolerance supported by regulatory cells is dominant and self-sustaining, there exists the possibility that cell-based immunotherapy may offer a curative option in diseases that would otherwise require long-term general immunosuppressive or anti-inflammatory therapy.

One particularly promising candidate cell type for use as an adjunct immunosuppressive agent in transplantation is the immunoregulatory macrophage (referred to herein and in the literature as "Mreg"). The Mreg cell reflects a unique state of macrophage differentiation, which is distinguished from macrophages in other activation states by its robust phenotype and potent T-cell suppressor function [5]. Human Mregs potently suppress mitogen-stimulated T-cell proliferation in vitro, which can be attributed to interferon (IFN) γ-induced indoleamine 2,3-dioxygenase activity, as well as contact-dependent deletion of activated T cells. In addition, Mregs drive the development of activated induced regulatory T cells that, in turn, suppress the proliferation of effector T cells and inhibit the maturation of dendritic cells. Therefore, when Mregs are administered to a recipient, it is hypothesized that a feed-forward loop of immunologic regulation is initiated leading to the long-term immunologic acceptance of a foreign transplant or prevention of immunopa-thology. Mreg-containing cell preparations have been administered to a total of 19 kidney transplant recipients as a form of adjunct immunosuppressive treatment in a series of case studies and two early-phase clinical trials [5]-[9]. These pilot studies clearly demonstrate the feasibility of this technique for solid organ transplantation.

An additional two living-donor kidney transplant recipients have now been treated with approximately 8.0×10⁶ cells/kg of purer donor-derived Mregs [5]. These two patients are now more than 6 years post transplantation with stable renal function on low-dose tacrolimus monotherapy as their sole maintenance immunosuppression. An additional clinical trial of Mreg therapy in living-donor renal transplantation now has regulatory approval within the framework of the ONE Study (Clinicaltrials.gov: NCT02085629). This trial aims to treat 16 patients with donor-derived Mreg cells at a dose of 2.5×10⁶ to 7.5×10⁶/kg body weight under cover of 500 mg/day mycophenolate mofetil on day 7 before surgery.

WO 2015/048641 A2 discloses a method of shifting macrophages towards an immunoregulatory M2 phenotype or inhibiting the formation of profibrotic macrophages by the addition of serum amyloid P (SAP). Ancuta et al. (2009): "Transcriptional profiling reveals developmental relationship and distinct biological functions of CD16+ and CD16- monocyte subsets", BMC GENOM-ICS, vol. 10, no. 1, page 403 describes the developmental relationship and distinct biological functions of CD16+ and CD16- monocyte subsets. The publication is concerned with monocytes and does not relate to regulatory macrophages. Hutchinson et al. (2011): "Human regulatory macrophages", Methods in Molecular Biology, vol. 677, pages 181-192 and Hutchinson et al. (2012): "Human regulatory macrophages as a cell-based medicinal product", Current Opinion in Organ Transplantation, vol. 17, no.1, pages 48-54 relate to the generation and characterization of immunoregulatory macrophages, involving cell culture of CD14+ monocytes with cell culture components such as M-CSF and interferon gamma. None of these publications, however, discloses the cultivation in bags.

Despite the great progress that was made in recent years in the field of immunoregulatory cells, there is an ongoing need for regulatory cells that can be used for therapeutic purposes, e.g. for inducing immunologic acceptance of a foreign transplant in a recipient, and for methods of preparing these cells in the utmost efficient way. In particular, there is a need for cell-based therapies that allow for the reduction of commonly used immunosuppressive medicaments which are regularly associated with a high degree of toxicity for the patient.

### SUMMARY OF THE INVENTION

The present invention provides a novel type of Mreg cell which significantly differs from Mregs that have been described before. It was found that a modified process for producing Mreg cells unexpectedly gave rise to a novel type of Mreg cell. Specifically, the inventors found that when the monocytic cells used for preparing the Mreg cells were cultured in gas-permeable bags instead of culture flasks, Mreg cells of a unique phenotype were obtained that exert immunoregulatory properties that render them highly suitable for cell-based therapeutic approaches. These cells are designated "Mregs-bc" herein to distinguish them over known Mregs.

Accordingly, in a first aspect the invention relates to a method for producing a novel type of macrophage which includes the culturing of monocytes from a blood sample of a subject in a gas-permeable bag in the presence of M-CSF, a CD16 ligand (such as an immunoglobulin), and IFN-Y.

In a second aspect, the invention refers to a novel type of Mreg cell, i.e. the Mreg-bc cell, which is obtainable by a method referred to in the first aspect of the invention. The Mreg-be cell has a unique phenotype which has not been observed in the prior art. The Mreg-be cell mediates biological activities that confer useful therapeutic properties which are unique to this cell type and have not been described in the prior art.

In a third aspect, the invention refers to a pharmaceutical composition comprising the Mreg-bc cell according to the second aspect of the invention. The pharmaceutical composition containing the new cell type of the invention may also contain further active ingredients or excipients as needed.

In a fourth aspect, the invention refers to the use of a Mreg-bc cell according to the second aspect of the invention or a pharmaceutical composition according to the third aspect of the invention for therapeutic purposes, in particular for the suppression of adverse immunological reactions.

Finally, in a fifth aspect, the invention refers to a process for preparing an immunoregulatory T cell by co-culturing T cells from a blood sample of a subject with an Mreg-bc cell according to the second aspect of the invention.

### BRIEF DESCRIPTION OF THE FIGURES

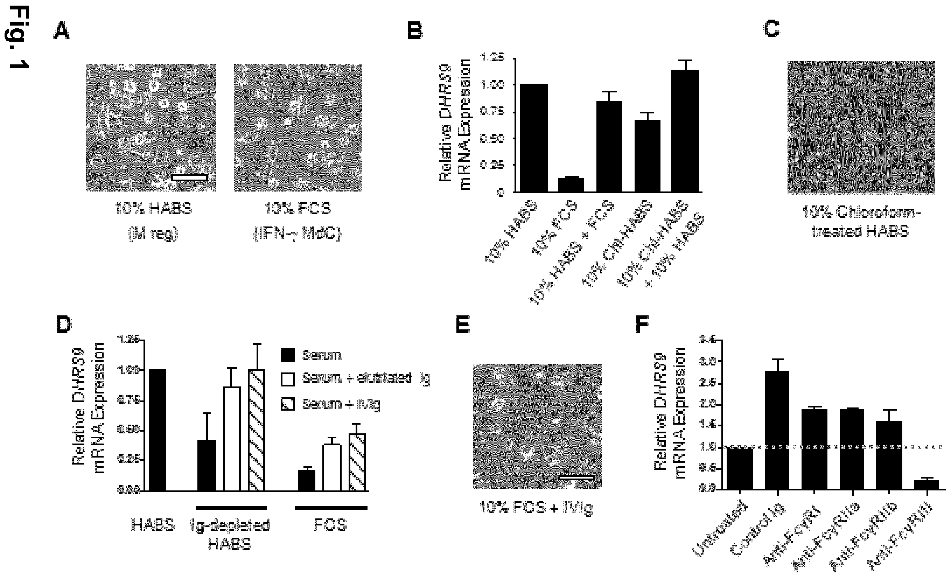

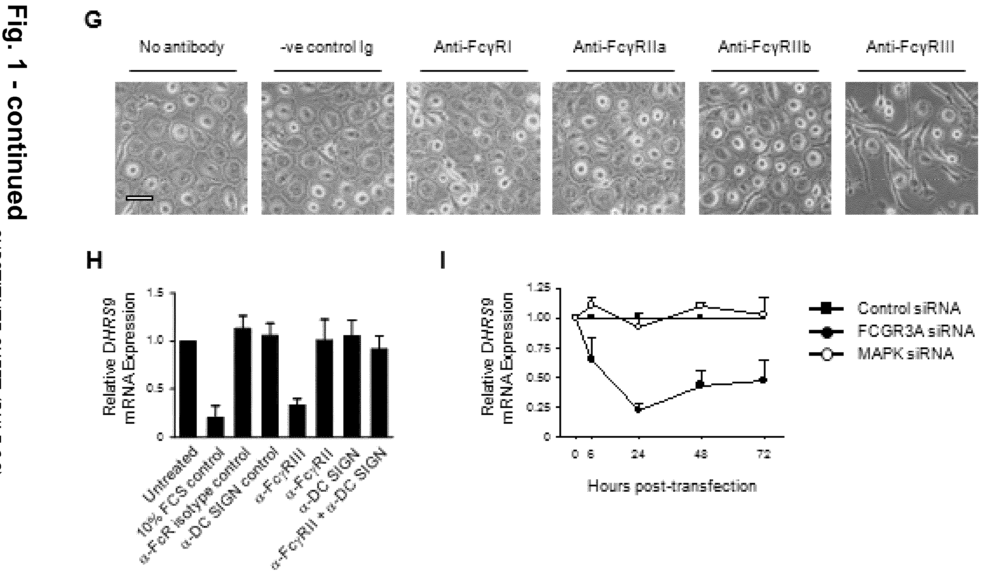

Figure 1 shows that Fc_{γ}RIII ligation drives the development of the Mregs. (A) Human Mregs generated in medium supplemented with 10% HABS exhibit a characteristic spreading morphology, whereas IFN-γ MΦ grown in medium containing 10% FCS, but under otherwise identical conditions, acquire an irregular, elongated form (bar = 50 µm). (B&C) After treatment of HABS with chloroform (Chl-HABS) its Mreg-inducing capacity was not lost, implying the existence of a non-lipid component of human serum responsible for Mreg development (n=6). (D) Culture of monocytes in Ig-depleted human serum decreased the expression of *DHRS9* mRNA compared to Mregs grown in 10% HABS (n=4). Addition of either Ig purified from serum or IVIG restored *DHRS9* mRNA expression. (D&E) DHRS9 mRNA expression by macrophages cultured in 10% FCS could be induced to some extent by both purified Ig and IVIg. (H) Antibody against Fc_{γ}RIII was most effective at preventing the HABS-induced expression of *DHRS9* mRNA by macrophages grown under Mreg culture conditions (n=5). (G) The addition of blocking antibody against Fc_{γ}RIII, but not other Fc_{γ}-receptors, prevented monocytes from acquiring Mreg morphology (bar = 50 µm). (H) Silencing Fc_{γ}RIII expression using siRNA confirmed the role of Fc_{γ}RIII in the induction of the Mreg phenotype by serum immunoglobulin. In all cases, bar graphs depict mean ± SEM.

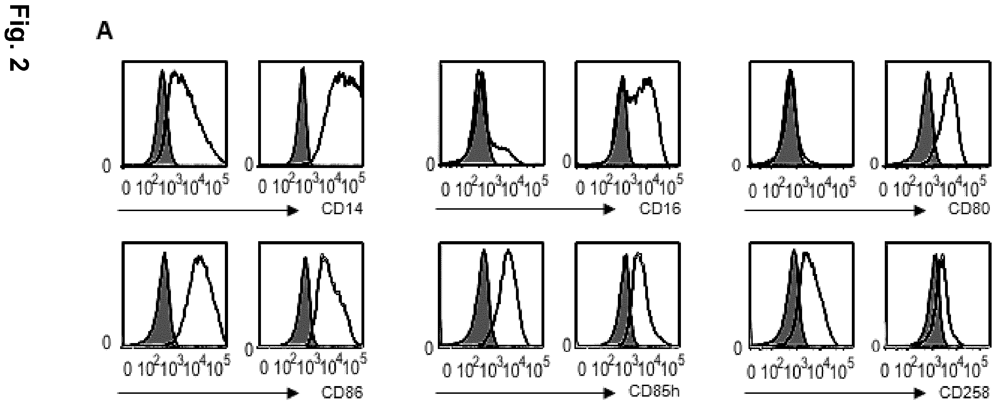

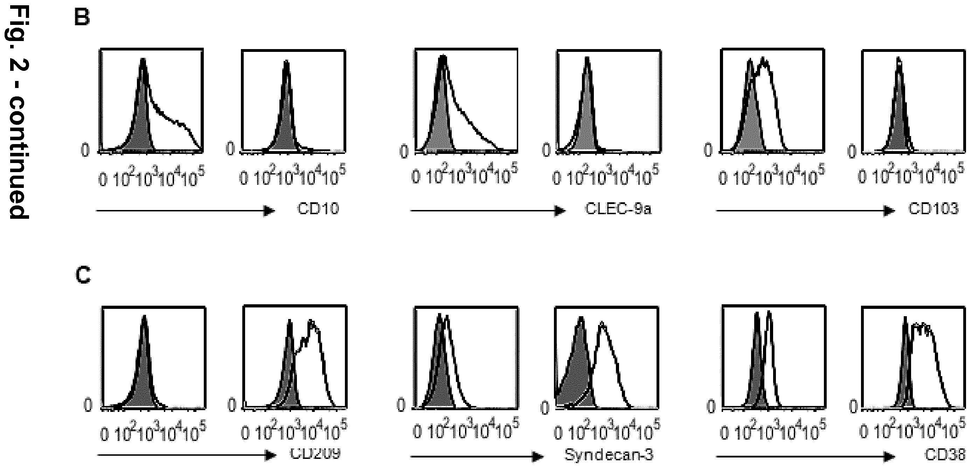

Figure 2 shows the comparison of the phenotype of Mregs cultured in flasks and Mregs-bc. (A) Mregs cultured in flasks (red traces) and Mregs-bc (blue traces) expressed the Mreg-defining cell-surface marker constellation of CD14^{-/low} CD16^{-/low} CD80^{-/low} CD86⁺ CD85h⁺ CD258⁺. Isotype control signals are shown in grey. (B) A proportion of Mregs-bc, but not Mregs cultured in flasks, expressed the antigens CD10, Clec9a and CD103. (C) Mregs cultured in flasks, but not Mregs-bc, expressed high levels of the antigens CD209, Syndecan-3 and CD38.

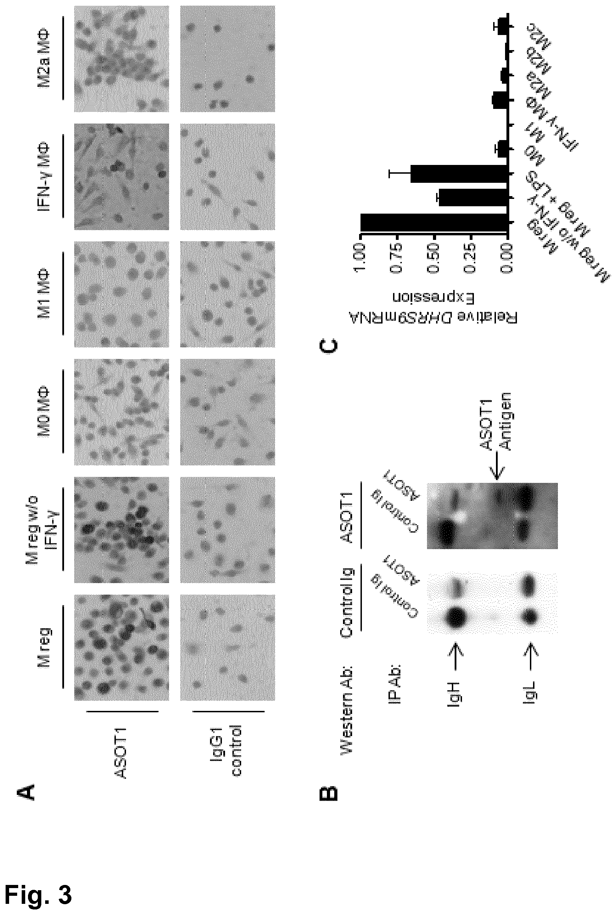

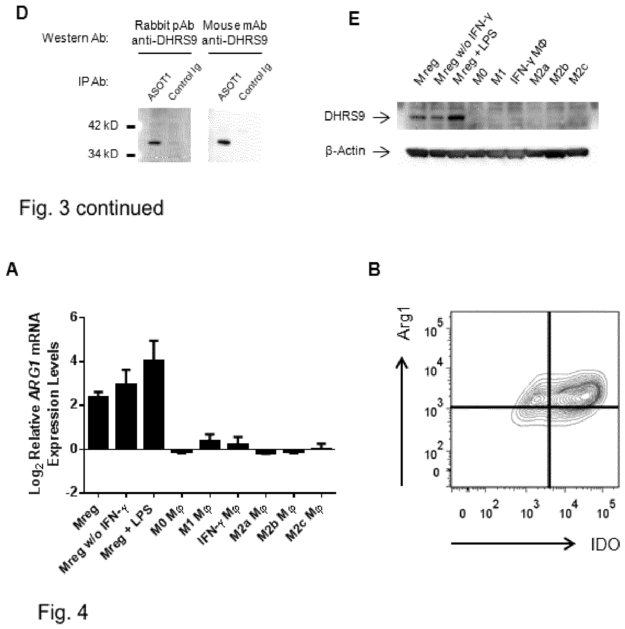

Figure 3 shows that dehydrogenase/reductase (SDR family) member 9 (DHRS9) expression uniquely identifies the Mreg phenotype. (A) The ASOT1 mAb recognised an antigen expressed by Mregs, but not other macrophage types. (B) An antigen of about 35 kD was specifically precipitated by ASOT1. This precipitated antigen was identified by mass spectrometry as DHRS9. (C) Strong DHRS9 mRNA expression was detected in Mregs, but not other macrophage types (n=6). (D) ASOT1 precipitated an antigen which was also recognised by an anti-DHRS9 rabbit pAb and mouse mAb, confirming that ASOT1 recognises DHRS9. (E) Immunoblotting with a rabbit anti-DHRS9 pAb demonstrated that DHRS9 expression at the protein level distinguishes Mregs from other macrophage types.
Figure 4 shows co-expression of IDO and Arginase-1 by Mregs. (A) Mregs, Mregs produced without IFN-γ stimulation, and lipopolysaccharide-treated Mregs expressed significantly higher levels of ARG1 mRNA than comparator macrophage types (n=3; mean ± SEM). (B) Flow cytometry staining reveals the coexpression of IDO and Arg1 by individual Mreg cells.

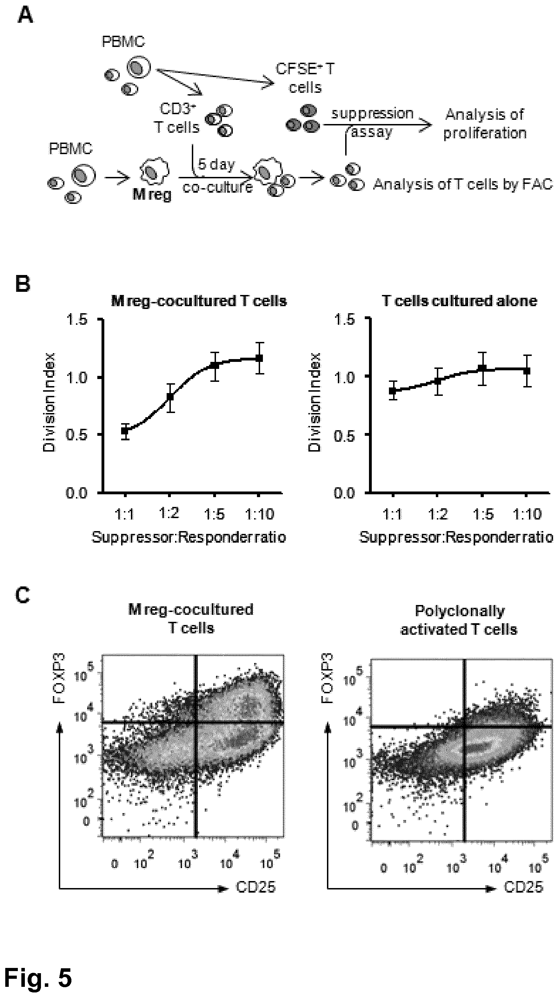

Figure 5 shows that T cells that have been co-cultured with Mregs-bc inhibit T cell proliferation. The functional consequences of exposure to Mregs-bc for allogeneic CD3⁺ T cells were investigated in co-culture experiments. (A) Human Mregs-bc generated from peripheral blood CD14⁺ monocytes were co-cultured in a 1:1 ratio with allogeneic CD3⁺ T cells isolated using CD3 microbeads (Miltenyi) in X-vivo 10 medium supplemented with 2 mM Glutamax and 25 ng/ml rhM-CSF for 5 days. Alternatively, Mregs-bc were co-cultured in a 1:1 ratio with allogeneic naive CD4⁺ T cells isolated using a naïve CD4⁺ T cell negative-isolation kit (Miltenyi) microbeads that were then incubated with CD3 microbeads (Miltenyi). After 5 days co-culture, T cells were re-isolated for phenotyping by flow cytometry. Using conventional methods for cell surface and intracellular staining, e.g. the Foxp3 fixation and permeabilization buffer kit (eBiosciences), co-cultured T cells are enriched for CD4⁺ CD25⁺ TIGIT⁺ FoxP3⁺ Tregs. Alternatively, re-isolated T cells are used as suppressor cells in an anti-CD3-stimulated, CFSE dilution-based allogeneic T cell proliferation assay. (B) Proliferation of CFSE-labeled responder CD4⁺ T cells stimulated with plate-bound anti-CD3 was inhibited to a greater degree by allogeneic Mreg-co-cultured T cells than by T cells that were cultured alone for 5 days (n=6; mean ± SEM). (C) CD3⁺ T cells co-cultured with allogeneic Mregs-bc for 5 days were enriched for CD25⁺ FoxP3⁺ Tregs, which were readily discriminated from CD25⁺FoxP3^{low} polyclonally activated T cells that had been stimulated with αCD3/αCD28 beads for 5 days. (Data representative of n=4 donor pairs.)

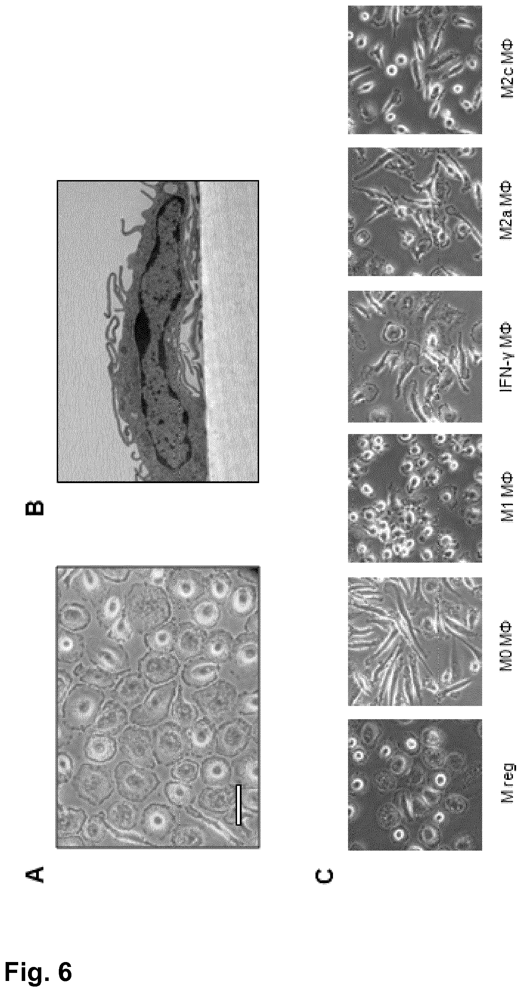

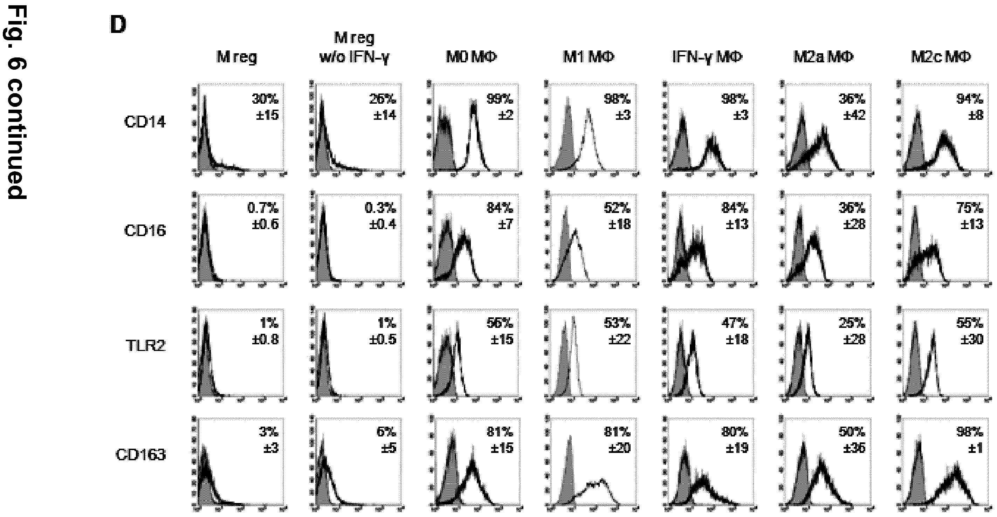

Figure 6 shows that Mregs exhibit a unique morphology and cell-surface phenotype. (A) Mregs in culture acquire a distinctive morphology (bar = 50 µm). (B) Transmission electron micrography of Mregs shows a close adherence to the culture surface, active nuclei with abundant fine chromatin, numerous cell processes and lipid inclusions. (C) Mregs are reliably distinguished from other macrophage polarisation states by their characteristic morphology in culture. (D) Mregs are distinguished from a panel of comparator macrophages by a constellation of cell surface markers: CD14^{-/low} CD16⁻ TLR2^{-/low} and CD163⁻ (n=6; mean ± SEM).

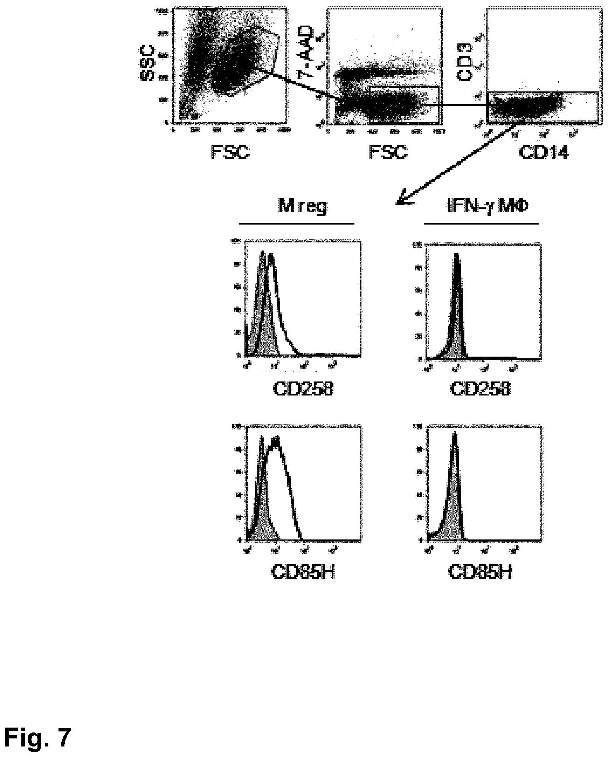

Figure 7 shows expression of CD85h (LILRA2; ILT1) and CD258 (TNFSF14; LIGHT), which were identified by microarray analyses as markers of human Mregs, are expressed at the cell-surface by Mregs but not by IFN-γ macrophages, as demonstrated by flow cytometry.

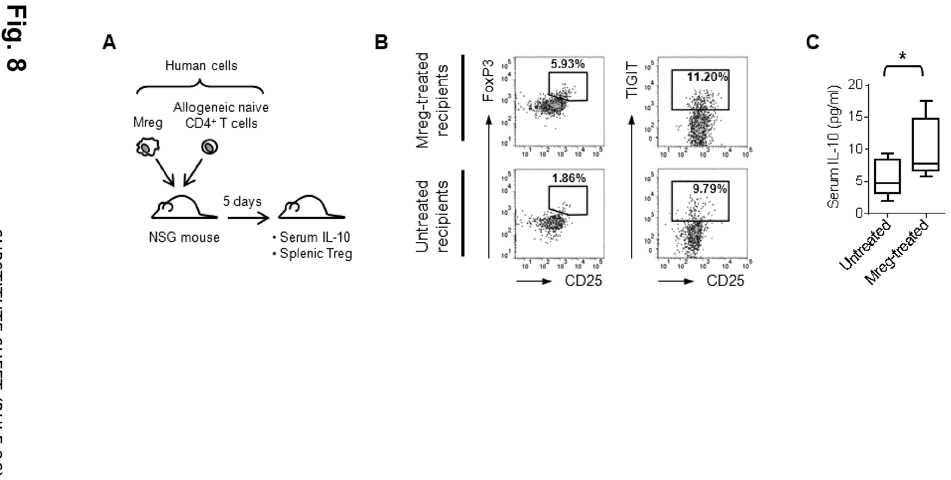

Figure 8 shows that Mregs-bc generate IL-10-producing TIGIT+ Tregs in vivo. (A) The ability of human Mreg-bc cells to induce TIGIT+ iTregs in vivo was investigated using an immunodeficient (NSG) mouse model. NSG mice received either (i) an i.v. injection of 5 × 10⁶ human naive CD4+ T cells alone or (ii) an i.v. injection of 5 × 10⁶ human naive CD4+ T cells plus a separate i.v. injection of 5 × 10⁶ allogeneic human Mregs. After 5 days, serum levels of human IL-10 and splenic human TIGIT+ Treg frequencies were assessed (n=12 donor pairs). (B) Mreg-treated recipients exhibited higher splenic Treg frequencies and somewhat higher TIGIT+ CD4+ T cell frequencies than untreated control animals. (C) Serum human IL-10 levels were significantly higher in Mreg-treated recipients than controls.

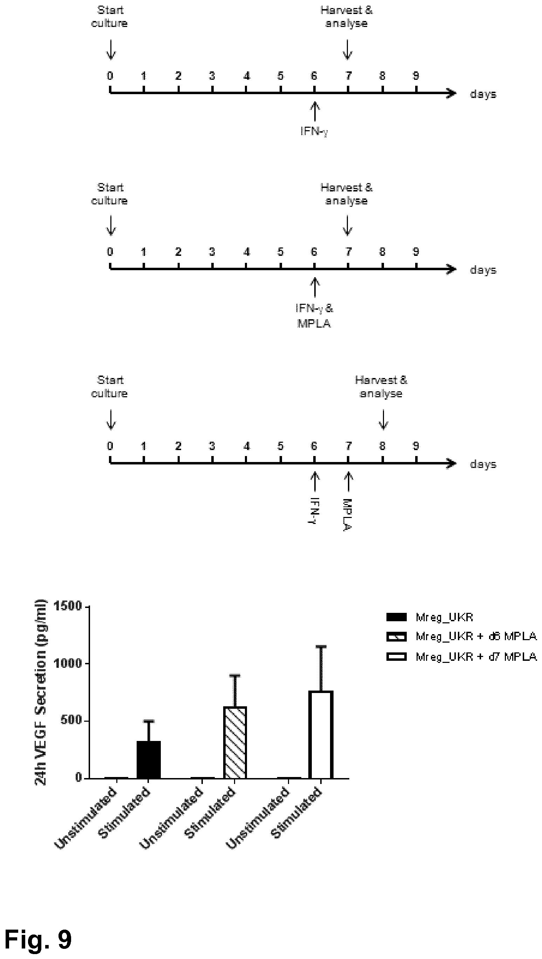

Figure 9 shows the set-up of an experiment for testing whether Mreg-bc cells produce angiogenic factor VEGF-A upon stimulation with monophosphoryl lipid A (MPLA).

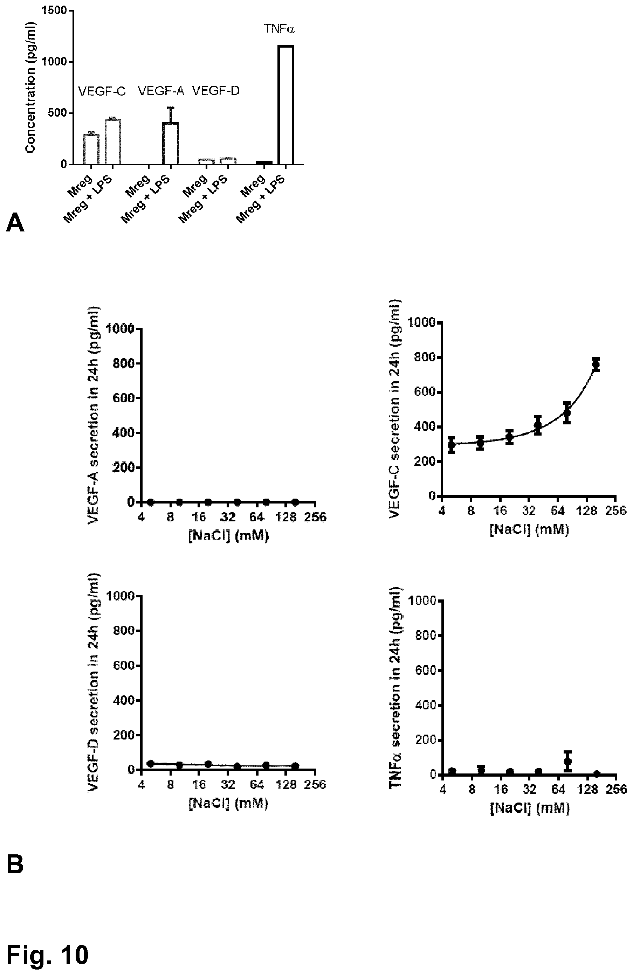

Figure 10 shows expression of Vascular Endothelial Growth Factor family members by Mregs upon stimulation with lipopolysaccharide (LPS) or under hypertonic culture conditions. (A) LPS stimulation elicited expression of VEGF-A, VEGF-C, but not VEGF-D; however, LPS stimulation also induced expression of TNF-α, a potent inflammory cytokine. (B) Mregs responded to increasing NaCl concentrations by secreting VEGF-C, but not VEGF-A or VEGF-D; critically, hypertonicity did not elicit TNF-α expression.

### DETAILED DESCRIPTION OF THE INVENTION

According to the invention Mreg-bc cells are derived from human CD14+ blood monocytes. In order to induce the characterizing biological properties of Mreg-bc cells, the monocytes are treated with a specific combination of growth factors, cytokines and receptor ligands. The cells obtained from the process of the invention are characterized by a unique phenotype that distinguishes them from blood monocytes, other types of monocyte-derived macrophages, monocyte-derived dendritic cells and other suppressive myelomonocytic cell products described in the prior art.

Accordingly, in a first aspect, the invention relates to a process for preparing a novel type of immunoregulatory macrophage cell, said process comprising:
(a) isolating CD14 positive monocytes from a blood sample of a subject;
(b) culturing the monocytes in a gas-permeable plastic bag in a culture medium containing (i) M-CSF and (ii) a ligand of CD16, wherein the concentration of M-CSF is in the range of 5-100 ng/ml;
(c) contacting the cells with IFN-y, wherein the concentration of IFN-γ is in the range of 5-100 ng/ml; and
(d) obtaining the immunoregulatory macrophage cell from the culture medium,
wherein the monocytes in step (b) are cultured for at least 5 days prior to IFN-γ stimulation.

The method of the invention uses blood monocytes as starting material. While it will be preferred that the method of the invention is used for generating Mreg-bc cells from human blood monocytes, the invention is not limited to the differentiation of cells of human origin. In fact, the invention is applicable also to other types of non-human cells, in particular vertebrate cells, e.g. non-human primate or pig cells. In this way, the invention provides an important contribution in the field of xenogenic transplantation medicine.

According to a preferred embodiment, the method of the invention is used to differentiate CD14 positive monocytes of a human donor into Mregs-bc. The monocytes which serve as a starting material for the method of the invention are obtained from the peripheral blood of a human donor. The donor may be a healthy subject or a patient suffering from one or more diseases. In one embodiment, the monocyte donor is the intended recipient of the differentiated Mreg-bc cells (autologous approach). In another embodiment, the monocyte donor is a separate person from the intended recipient of the differentiated Mreg-bc cells (allogeneic approach). In the latter case, the donor and recipient may be genetically related or unrelated. In another embodiment, the monocyte donor is a separate person from the intended recipient of the differentiated Mreg-bc cells, but is also the donor of other cells, tissues or organs for transplantation into the same recipient. The preferred relationship between donor and recipient depends upon the clinical application. The use of autologous Mreg-bc cells may help to avoid certain adverse reactions. Therefore the use of autologous Mreg-bc cells is preferred in the case of regenerative or anti-inflammatory therapies. **In** the transplant setting, the use of donor-derived Mreg-bc cells as immunosuppressive therapy is preferred because donor antigen-expressing cells are more effective than recipient-derived cells [11].

Different methods are known in the art for the enrichment of mononuclear cells from peripheral blood, and each of these methods can be used in the context with the present invention. For example, blood obtained by venepuncture can be treated with an anticoagulant and subsequently separated by use of a separation medium, such as Ficoll-Paque Plus. For this, the anticoagulant-treated blood sample is layered on the Ficoll-Paque Plus solution and centrifuged, which will result in the formation of layers containing the different cell types. The bottom layer contains erythrocytes which have been aggregated and sedimented by the Ficoll-Paque Plus reagent. The layer immediately above the erythrocyte layer contains mostly granulocytes which have migrated through the Ficoll-Paque Plus layer. Owing to their lower density, monocytes and lymphocytes are found at the interface between the plasma and the Ficoll-Paque Plus. Enrichment of the mononuclear cell fraction can be achieved by isolation of the layer and subsequent washing and centrifugation.

Another routinely used method for separating mononuclear leucocytes from blood samples includes leukapheresis. Leukapheresis is a specific type of apheresis in which white blood cells are obtained from peripheral blood according to their relative densities in a continuous process. In this procedure, the blood of a subject is passed through a special centrifugation device which collects the chosen fraction of white blood cells and returns the remaining blood cells and plasma back to the donor. Leukapheresis is nowadays a routine clinical measure for obtaining leucocytes or stem cells from peripheral blood. Different devices are available from several manufactures that can be used for performing leukapheresis in the context with the present invention, e.g. the COBE^{®} Spectra Apheresis System from Terumo BCT. Where the leukapheresis is carried out by use of the COBE^{®} Spectra Apheresis System, it is preferred to use the manual protocol provided by the manufacturer, since this protocol was found to result in better quality monocytes compared to the AutoPBSC protocol.

Both the use of a separation medium like Ficoll-Paque Plus and the use of a leukapheresis device will provide a cell fraction that contains, apart from the monocytes, also lymphocytes. According to the invention, monocytes may be enriched and separated from the lymphocytes by known methods, e.g. by magnetic bead separation, sorting by flow cytometry, elutriation, filtration or plastic adherence, before the cells are introduced into the preparation method of the present invention. However, it is not mandatory to use a homogeneous monocyte fraction in the method of the invention. In fact, the presence of an amount of 0.1-20%, preferably 10-20% lymphocytes in the monocyte fraction may positively influence the differentiation of monocytes to regulatory macrophages.

In one embodiment of the invention, the monocyte fraction used in the method of the invention is essentially pure and contains less than 15%, less than 10%, less than 5%, less than 4%, less than 3%, less than 2%, or less than 1% percent of non-monocytic nucleated blood cells, such as lymphocytes or granulocytes. For obtaining a mononuclear cell preparation that is enriched for monocytes, peripheral blood mononuclear cells may be contacted, e.g., with CD14 microbeads to which CD14-positive monocytes bind. In one embodiment, the monocytes in step (a) are isolated by leukapheresis and subsequent subjected to a separation step using CD14 affinity molecules, preferably CD14 antibodies. Such a purification step massively reduces contamination of the starting material with non-monocytes. Reduction of T cell contamination is very valuable from a patient safety perspective because it minimizes the potential risk of donor-versus-recipient reactions. In a preferred embodiment of the invention, the CD14 monocytes which are used in the method of the invention have been isolated with the CliniMACS^{®} Technology (Miltenyi Biotec GmbH, Bergisch Gladbach, Germany).

The monocyte fraction, which has been isolated by leukapheresis and/or other methods, can directly be used for differentiation by incubation with M-CSF and the CD16 ligand, or it can be stored in autologous plasma supplemented with Anticoagulant Citrate Dextrose Solution (ACD-A) or any other suitable buffer until further use. If the isolated monocyte fraction has to be transported to a different site where the differentiation process is carried out, care should be taken that differentiation of the cells by incubation with M-CSF is started within 24 hours after isolation of the cells, preferably within 18 hours, within 12 hours, within 6 hours, within 4 hours, or within 2 hours after isolation of the monocytes. For long term storage, the monocyte fraction may be re-suspended in a suitable cryopreservation solution and stored at temperatures below 20°C, preferably below 80°C for extended periods of time.

After isolation of the monocytes, the cells are incubated in the presence of M-CSF and a CD16 ligand. For example, the cells may be suspended in a medium that contains M-CSF and a CD16 ligand. Alternatively, it is also possible to add M-CSF and the CD16 ligand sometime after start of cell culturing. The culture medium used in step (b) of the above method can be any medium that has been described in the literature as suitable for use in the culturing of monocytes and/or macrophages. Suitable culturing media include, for example, the PromoCell Macrophage Generation Medium (PromoCell GmbH, Heidelberg, Germany), Dulbecco's modified Eagle's medium (DMEM), DMEM:F12 blend, Medium 199, or RPMI-1640 medium. The culture medium preferably is a chemically defined medium. Apart from M-CSF, the culture medium can contain other factors to promote the survival and differentiation of Mregs, including: growth factors and cytokines, such as epidermal growth factor (EGF), or IL-4; fatty acids, cholesterol and other lipids; vitamins, transferrin and trace elements; insulin, glucocorticoids, cholecalciferol or ergocalciferol, and other hormones; non-specific immunoglobulin and other plasma proteins. In a preferred embodiment of the invention, the culture medium is RPMI-1640 or a medium derived therefrom.

The culture medium used for incubating the isolated CD14 positive monocytes contains Macrophage Colony-Stimulating Factor (M-CSF, also known as CSF1). M-CSF is known in the art as a hematopoietic growth factor that influences the proliferation, differentiation, and survival of monocytes, macrophages, and bone marrow progenitor cells. Granulocyte-macrophage colony-stimulating factor (GM-CSF, also known as CSF2), is a monomeric glycoprotein that functions as a cytokine and is secreted by macrophages, T cells, mast cells, NK cells, endothelial cells and fibroblasts. M-CSF and GM-CSF proteins from different species have been described and can be purchased from different manufacturers. The choice of the M-CSF used in the method of the invention will depend on the origin of the monocytes which are to be differentiated into Mreg-bc cells. For example, if human monocytes are differentiated to Mregs-bc using the process described herein, the medium used will contain human M-CSF, preferably recombinant human M-CSF. Similarly, if porcine monocytes are used in the differentiation method, the M-CSF added to the medium will be of porcine origin. In a particularly preferred embodiment of the invention, the M-CSF is of human origin, such as recombinant human M-CSF, and the monocytes are human monocytes.

The skilled person will be able to find an amount of M-CSF which is suitable for differentiating a high proportion of the monocytes into Mregs-bc by routine methods. According to the invention, the concentration of M-CSF in the culture medium in step (b) of the above method is in the range of 5-100 ng protein per ml medium. Time-course experiments to measure the amount of M-CSF in the culture medium revealed that M-CSF was consumed or degraded over time, such that cultures with an initial dose of 5 ng/ml M-CSF contained sub-physiological concentrations by day 2 of culturing; in contrast, cultures with an initial dose of 25 ng/ml M-CSF maintained concentrations of >10 ng/ml throughout a 7-day culturing period. Thus, in a preferred embodiment of the invention, the concentration of M-CSF in the culture medium is in the range of 20-75 ng/ml, 20-50 ng/ml or 20-25 ng/ml. A concentration of at least 25 ng M-CSF per ml culture medium is particularly preferred. Preferably, the above concentrations refer to recombinant human M-CSF.

In cases where both M-CSF and GM-CSF are used in the medium, the overall concentrations of these two growth factors will be in the above-mentioned range, i.e. in the range of 20-75 ng/ml, 20-50 ng/ml or 20-25 ng/ml. An overall concentration of M-CSF and GM-CSF of 25 ng M-CSF per ml culture medium is particularly preferred.

Apart from the M-CSF , the culture medium used in step (b) of the above method also comprises a CD16 ligand. It has been found that stimulation of the CD16 cell surface receptor on the monocytes is required to induce their differentiation into Mreg-bc cells. More specifically, experiments conducted in the course of the present invention revealed that monocytes grown in medium supplemented with human AB serum (HABS) develop into Mregs, whereas monocytes grown in medium supplemented with fetal calf serum (FCS) do not develop into Mregs. Monocytes grown in an equal mixture of both sera develop the Mreg-phenotype. Therefore, HABS contains a positive Mreg-inducing activity (see Figure 1A&B). Removal of the chloroform-extractible fraction of HABS demonstrated that the Mreg-inducing activity of HABS principally resided within the chloroform-resistant fraction, so was likely to be a protein (see Figure 1B&C). By size-fractionation, the principal protein component of HABS responsible for Mreg-be development was found to be >100 kDa leading to the hypothesis that the unknown factor was immunoglobulin (Ig). HABS depleted of Ig using protein A/G sepharose was unable to support the development of Mreg-bc morphology and DHRS9 mRNA expression (see Figure 1D). Re-addition of elutriated Ig back into the Ig-depleted serum (or addition of IVIg) restored its ability of to induce DHRS9 expression (see Figure 1D). Similarly, when monocytes were cultured in FCS supplemented with human Ig, an increase in the *DHRS9* mRNA expression was observed compared to FCS-alone controls and normal Mreg-bc morphology was obtained (Fig. 1D&E). Monocytes treated with anti-Fc_{γ}RIII antibody expressed significantly lower levels of *DHRS9* mRNA than monocytes treated with anti-Fc_{γ}RI (CD64), anti-Fc_{γ}RIIa/b (CD32a/b) or control antibody (see Figure 1F) and did not develop Mreg-bc morphology (see Figure 1G). Blockade of either Fc_{γ}RIIb or DC-SIGN alone, or both receptors together, had no effect on the generation of DHRS9⁺ Mregs (see Figure 1H). To reinforce the observation that Fc_{γ}RIII is necessary for Mreg-bc generation, Fc_{γ}RIII expression was silenced using siRNA (see Figure 1I). A transient suppression of FCGR3A and FCGR3B transcript expression was achieved in freshly isolated monocytes cultured in 10% HABS; importantly, FCGR2B expression was not decreased by this manipulation. Knockdown of Fc_{γ}RIII at the protein level was demonstrated by flow cytometry (35.2% ± 4.4 CD16⁺ cells using negative control siRNA, versus 15.3% ± 3.7 with FCGR3 siRNA; n=4, p=0.002). Silencing Fc_{γ}RIII expression (but not suppression of MAPK1 expression or treatment with a negative-control siRNA) resulted in a significant down-regulation of DHRS9 mRNA expression (see Figure 1I).

It was concluded from the above findings that serum Ig acts through Fc_{γ}RIII (CD16) to induce the Mreg phenotype. The dependence of Mreg differentiation on Fc_{γ}RIII distinguishes the Mreg from other Ig complex-induced macrophage types described in the prior art. In particular, the mode of derivation distinguishes the Fc_{γ}RIII-induced Mreg from the Fc_{γ}RIIb-induced macrophage, Fc_{γ}RI-induced macrophage and macrophages generated in the absence of immunoglobulin which were described in the prior art.

As stimulation of the CD16 cell surface receptor is crucial for the differentiation into the desired Mreg-bc phenotype, the method of the invention includes the incubation of the monocytes with a CD16 ligand in step (b). The ligand which binds to the receptor will preferably be a human or non-human immunoglobulin, and more preferably a human immunoglobulin, or a fragment thereof. The immunoglobulin fragment can be, for example, an Fc fragment of an immunoglobulin. The immunoglobulin or immunoglobulin fragment is preferably added to a serum-free culture medium. Alternatively, recombinant proteins may be used which comprise a sequence of an immunoglobulin or immunoglobulin fragment, such as a sequence of a human immunoglobulin. In another embodiment, a non-human or human antibody or a fragment thereof which specifically binds to CD16 through its antigen recognition domain is used to promote Mreg-bc differentiation. In still another embodiment, small molecules are used to stimulate the CD16 signaling pathway to promote Mreg-bc differentiation.

In a preferred embodiment, the medium used for generating the Mreg-bc cells contains 1-20% human serum or equivalent amounts of certain serum components, such as immunoglobulin. More preferably, the medium is supplemented with 10% serum. If serum-containing media are used for carrying out the method of the invention, the media comprise between 5-15%, preferably 10%, human serum. A medium containing 10% human AB serum is particularly preferred. Stated differently, it is preferred that the serum is added in a concentration of about 0.01 to 10 mg/ml, preferably about 0.1 to 1 mg/ml, and more preferably about 1 mg/ml. Slightly lower concentrations can be used when using immunoglobulin or immunoglobulin fragments as CD16 ligands. Substantially lower concentrations may be used if immunoglobulin or other CD16 ligands are immobilized on tissue culture surfaces, beads or other physical matrices. It is further preferred that the human serum, such as AB serum, is derived from male donors. Where serum is used from female donors, care should be taken that the donors do not use progesterone or progesterone-oestrogen contraceptives. It is further preferred that said medium should not contain antibodies against monocytes or Mreg-be cells or any intermediate form, including antibodies against major histo-compatibility molecules.

It was also found that antibiotics in the culture medium had no measurable effect on the viability, yield, phenotype or suppressive function of the Mregs-bc produced by the method of the invention. Accordingly, it is preferred that the medium used in step (b) of the method of the invention does not contain any antibiotic.

Where the Mregs-bc cells are intended for use in therapeutic applications in which the induction of angiogenesis is desired (see below), the medium used for culturing the monocytes in step (b) of the method of the invention may comprise, apart from M-CSF and the CD16 ligand, a toll-like receptor (TLR) ligand, such as lipolysaccharide (LPS), monophosphoryl lipid A (MPLA) or High Mobility Group Box protein 1 (HMGB1) to enhance the production of angiogenic factors like VEGF-A. The TLR ligand can be added to the culture medium in a concentration range of 1000 ng/ml to 1 µg/ml, preferably between 50-500 ng/ml, such as 100 ng/ml, 200 ng/ml, 300 ng/ml, or 400 ng/ml. In cases where more than one TLR ligand is added, the overall concentration of these ligands should be in the above-recited range. The TLR ligand can be added at any stage of the production method. It can be present in the initial medium which is used for culturing the monocytes, i.e. at day 0 of the culture, or it can be added at a later stage, e.g. at day 5, 6 or 7 of the culture. Preferably, the TLR ligand is added simultaneously with the addition of the IFN-γ.

According to the invention, the method for preparing the Mreg-bc cells includes culturing of the monocytes in the presence of M-CSF and the CD16 ligand in a gas-permeable bag. Once the monocytes have been suspended in a suitable medium, the cell suspensions are transferred to gas-permeable plastic bags for culturing and differentiation. Bags for cell culturing are available from different suppliers, for example from Miltenyi Biotec GmbH (Bergisch Gladbach, Germany), Thermo Fisher Scientific (Schwerte, Germany) or Merck (Darmstadt, Germany). The bags will be made of a plastic material that allows the attachment of the cultured cells to the inner surface of the culturing bag. Bags consisting of polyolefine or polyethylene are preferred.

The bags will preferably be designed to allow a cell plating density of 1-2 × 10⁶ monocytes per cm² internal culture surface. This means that a cell suspension containing 180 × 10⁶ monocytes is preferably cultured in a bag that has an internal surface area of at least 90 cm² and not more than 180 cm². The optimal density of cells in suspension preferably is between about 1 × 10⁵ cells/ml and 1 × 10⁷ cells/ml, and more preferably 1 × 10⁶ cells/ml. The ratio of cell suspension volume to bag volume is at least 1.0, preferably 0.2 and more preferably 0.06 in order to minimise the amount of medium from which Mregs-bc must be concentrated at the end of culture. This means that a 3L culture bag will be filled with 1L cell suspension or less, preferably 600 ml or less, and more preferably 180 ml or less. In a preferred embodiment of the method of the invention, the volume of the bags used for culturing the monocytes in the medium that has been supplemented with M-CSF and the CD16 ligand is at least 3 L.

After the monocytes have been transferred to the culture bags, the cells are incubated in the bags in the presence of M-CSF and the CD16 ligand, e.g. human serum or human immunoglobulins, for at least 5 days prior to IFN-γ stimulation. As used herein, a culturing period of "1 day" refers to 24 hours of culturing. Accordingly, a culturing period of "at least 3 days" refers to 72 hours of culturing or more. The optimal period of IFN-γ stimulation is at least 12 hours, preferably 18 hours, and more preferably 24 hours. According to the invention, the total culturing period, i.e. the time period from introducing the monocytes into the culturing bags to harvesting of the Mregs-bc is at least 5 days, at least 6 days, at least 7 days or at least 8 days. Stated differently, the total culturing period is between 5 and 8 days, preferably between 6 and 8 days, more preferably 7 days. The monocytes in the culture bags are incubated under conditions that allow for their growth and differentiation into Mreg-bc cells. The general conditions for culturing monocytes or macrophages are known to a person working in the field of cell culturing.

For example, the bags containing the suspensions can be transferred to an incubation chamber which allows the selection of defined conditions of temperature, humidity and CO₂. Suitable conditions a temperature in the range of 30-40°C, preferably between 32°-38°C, and more preferably between 37-38°C, e.g. 37°C. The humidity used for culturing is normally in the range of 30-70%, preferably 40-60%, and more preferably 50-60%, e.g. 60% humidity. The incubation chamber may include up to 10% CO₂. A content of up to 5% CO₂, up to 4% CO₂, up to 3% CO₂, up to 2% CO₂, or up to 1% CO₂ is particularly preferred. The bags are preferably laid flat on a shelf of the incubation chamber during incubation.

The monocytes in the bags are preferably intermittently softly agitated to allow their semi-adherent attachment to the lower leaf of the culture bag. It is preferred that the bags are inverted at least once within the total culturing period so as to allow their adherence to the opposite leaf of the bag. In another embodiment, the bag is inverted at least twice during the total culturing period. In another embodiment, the bag is inverted at least three or four times during the total culturing period. In a still further embodiment, the bag is inverted every 24 hours during the total culturing period. In another embodiment, the bag is inverted every 36 hours during the total culturing period. In yet another embodiment, the bag is inverted every 48 hours during the total culturing period.

In step (c) of the method of the invention, the cells are contacted with the cytokine interferon gamma (IFN-y). The cytokine is known in the art to alter the transcription of more than 30 genes, thereby producing a variety of physiological and cellular responses. IFN-γ proteins have been isolated from different species and can be purchased from different manufacturers. The choice of the IFN-γ used in the method of the invention will depend on the origin of the monocytes which are subjected to the method of the invention. For example, if human monocytes are differentiated to Mregs-bc using the process described herein, the IFN-γ added will be human IFN-γ, preferably recombinant human IFN-γ. Similarly, if porcine monocytes are used in the differentiation method, the IFN-γ added to the medium will be of porcine origin. In a particularly preferred embodiment of the invention, the IFN-γ is human IFN-γ, more preferably recombinant human IFN-γ.

Any amount of IFN-γ may be added that is effective to induce the expression of indoleamine 2,3-dioxygenase (IDO) by the monocytes in the culture. The amount of IFN-γ to be added to the monocyte culture is in the range of 5-100 ng/ml, more preferably between 10-80 ng/ml, still more preferably between 20-50 ng/ml. An amount of 25 ng IFN-γ per ml culture medium is particularly preferred herein.

The IFN-γ can be added to the medium simultaneously with the M-CSF and the CD16 ligand which means that the cytokine may be added, e.g., at the time when the monocytes are introduced into the culturing bags. In such an embodiment, the monocytes to be differentiated by the method of the invention will be cultured in the presence of M-CSF, the CD16 ligand and IFN-γ for the entire culturing period. It is however preferred that the culturing period in the presence of IFN-γ is considerably shorter than the culturing period in the presence of M-CSF, which means that the IFN-γ is added only after the cells have been cultured for at least 5 days in the presence of M-CSF Preferably, the cells have been cultured for at least 6 days in the presence of M-CSF before the addition of IFN-γ. In a particularly preferred embodiment, the IFN-γ is added after having cultured the cells for 5-6 days in the presence of M-CSF, and culturing is then continued for another 18-72 hours.

In a particularly preferred embodiment of the invention, the differentiated cells are harvested at day 7, e.g. after 6 days of culturing the monocytes in the medium containing M-CSF and the CD16 ligand followed by 18-24 hours IFN-γ stimulation. Where several bags have been cultured in parallel, the content of the bags may be pooled at the end of the culturing process. The differentiated macrophages may be washed by a buffer which is compatible for use with macrophages. For example, Ringer solution or Phosphate Buffered Saline (PBS), preferably supplemented with 5% human serum albumin, can be used for washing the cells by serial exchange of the buffer by centrifugation and decanting the supernatant. It has been found in the course of the present invention that the use of trypsin does not improve the yield of immunoregulatory macrophages. Therefore, it is preferred herein that the harvesting step does not include the addition of trypsin.

These Mreg-bc cells can be transferred and stored in a transfusion bag, a glass infusion device or in another closed-system container which allow for the transportation of the cells to the treatment center or to the patient's bedside. For this purpose, the differentiated cells will be suspended in a suitable preservation medium. The preservation medium can be, for example, Ringer solution, which is preferably supplemented with 5% human serum albumin. In a particularly preferred embodiment, the preservation medium is a ready-to-use medium which is serum-free and/or protein-free. A suitable ready-to-use medium which is commercially available is HypoThermosol^{®} FRS (Stemcell Technologies SARL, Köln, Germany). Preferably, the medium has a pH of between 6.5 and 8.0, more preferably between 7.0 and 7.5, such as 7.4. The cell solution should be stored at 4°C to minimize energy consumption and cell adhesion. Alternatively, Mreg-bc cells may be re-suspended in cryopreservation solution and stored in a frozen form until final use.

The phenotypic and functional stability of the differentiated macrophage cells of the invention depends upon the choice of excipient and storage temperature. When resuspended in Ringer solution supplemented with human serum albumin, the macrophages of the invention are stable at 20°C to 25°C for up to 24 hours after cell harvest. When resuspended in HypoThermosol^{®} FRS, the macrophages can be stored at 2-8°C, preferably 4°C, for at least 72 hours after cell harvest. When longer storage periods are required, the cells may be subjected to freezing or cryopreservation. Generally, it was found that the cells of the invention are stable in their immunosuppressive phenotype. A treatment with pro-inflammatory mediators, e.g. lipopolysaccharide, does not drive them to develop a stimulatory phenotype.

The method for preparing Mreg-bc cells of the invention can be automatized according to common methods, e.g. by using a GMP-compliant platform which offers integrated solutions that streamline cell-processing workflows. The process preferably occurs in a "closed system" which takes advantage of closed disposables, optional customization of tubing sets, buffers and reagents, multiple input lines with sterile filters, output line for optional in-process controls and substantially reduced clean room requirements. For example, the platform may comprise a cell separation system enabling the separation of monocytes from the white blood cell fraction. The cell separation system should be able to separate monocytes from a starting volume of 100-1000 ml aphere-sate or whole peripheral blood. The monocytes contained in the isolated mononuclear white blood cell fractions may then be isolated, e.g. via magnetic beads, that bind to CD14+ cells. These cells are then cultured in an appropriate culture medium. The platform allows providing media, growth factors and/or cytokines to the cell culture via multiple input ports. At the end of the culturing process, the cells are automatically washed, harvested and transferred into appropriate sterile delivery bags. A customized tube sealer may be used that permits sterile sealing of PVC and EVA tubes. The cellular product may be bar-coded, and the whole manufacturer process may be monitored online for quality control purposes.

In a second aspect, the invention refers to a novel type of Mreg cell, referred to as Mreg-bc cell, which is obtainable by the method of the first aspect of the invention. The cells provided by the invention are monocyte-derived human macrophages and, as such, express common leukocyte markers and macrophage lineage markers, in particular CD45, CD11b, CD33 and HLA-DR. Mregs are distinguished from monocytes, a panel of comparator macrophages (i.e. resting, M1, M2a, M2b and M2c macrophages) and monocyte-derived DCs by a constellation of lineage and activation markers - namely, CD14^{-/low} CD16^{-/low} CD80^{-/low} CD86⁺ CD85h⁺ CD258⁺ (see Figure 2A). CD85h is expressed in Mregs and monocytes, but its expression is lost in resting macrophages, M1 macrophages, M2a macrophages, M2b (Ig complex-stimulated) macrophages, M2c (dexamethasone-treated) macrophages, and monocyte-derived dendritic cells. CD258 is expressed in Mregs and M2b macrophages, but it is not expressed in monocytes, resting macrophages, M1 macrophages, M2a macrophages, M2c (dexamethasone-treated) macrophages, and monocyte-derived dendritic cells.

Comparing Mregs-bc, i.e. cells cultured in bags, with Mregs cultured under otherwise identical conditions in flasks, Mregs-bc consistently express lower levels of CD14, CD16 and CD80 than flask-cultured cells. Mregs-bc consistently expressed higher levels of CD85h and CD258 than flask-cultured Mregs. Mregs-bc can be distinguished from those that have been cultured in flasks by the expression of the markers Clec-9a, CD10 and CD103 (see Figure 2B). Alternatively, in contrast to common Mregs, Mregs-bc do not express (or express only in low amounts) the markers CD38, CD209 and Syndecan-3 (see Figure 2C). Characteristically, all human Mregs, either bag-cultured or flask-cultured, express relatively high levels of DHRS9, a retinol dehydrogenase of the SDR family of retinol dehydrogenases (see Figure 3). Single human Mreg cells, either bag-cultured or flask-cultured, concomitantly express both indoleamine 2,3-dioxygenase (IDO) and Arginase-1 (Arg1) which is not observed in other monocyte-derived macrophage types described in the prior art (see Fig. 4).

Accordingly, the invention provides an Mreg-bc cell obtainable by the process of the invention described above that does not express one or more of the markers CD38, CD209 and Syndecan-3 (or expresses these markers only at a low level). Herein, a cell is negative for a particular surface marker if its fluorescence intensity as measured by flow cytometry is less than the fluorescence intensity of the 99th percentile of a corresponding isotype control-stained sample. Preferably, Mreg-bc cells of the invention are negative for CD209. The Mreg-bc cells are furthermore either negative for CD38, or they express low levels of CD38. During the generation of Mreg-bc cells, the initial population of monocytes downregulates CD38 expression at the cell surface. Down-regulation of CD38 during Mreg-bc development can be expressed as the percentage of CD38 expression on Mregs-bc at day 7 compared to monocytes on day 0 (d0) of culture. The expression of CD38 is proportional to the difference in mean fluorescence intensity between an isotype control-stained cell and the specific CD38 signal. Hence, % down-regulation = 100 - 100 × (CD38_{d7} - Iso_{d7})/(CD38_{d0} - Iso_{d0}), wherein CD38_{d7} is the specific signal on day 7; Iso_{d7} is the isotype control signal on day 7; CD38_{d0} is the specific signal on day 0; Iso_{d0} is the isotype control signal on day 0. The down-regulation of CD38 by Mregs-bc cells can be conveniently determined by using standard flow cytometry methods. According to a preferred embodiment, the expression of CD38 by the Mreg-bc cell is downregulated by more than 50% relative to the initial expression of CD38 by monocytes on day 0 of culture, more preferably by more than 60%, by more than 70%, by more than 80%, by more than 90%, by more than 95% or by more than 99%.

Similarly, during the generation of Mreg-bc cells, the initial population of monocytes acquires a low-level of Syndecan-3 expression at the cell surface, whereas Mreg cells cultured in flasks acquire a higher level of Syndecan-3 expression at the cell surface. Therefore, the differentiated Mreg-bc cells obtained from the method of the invention do not express the marker Syndecan-3 or does only express the marker at a comparatively low level. Expression of Syndecan-3 by Mreg-bc cells can be expressed in relation to Syndecan-3 expression on flask-cultured Mregs. The expression of Syndecan-3 is proportional to the difference in mean fluorescence intensity between an isotype control-stained cell and the specific Syndecan-3 signal. Hence, % expression = (Syndecan-3_{Mreg-bc} - Iso_{Mreg-bc})/(Syndecan-3_{flask} - Iso_{flask}), wherein Syndecan-3_{Mreg-bc} is the specific signal of Syndecan-3-stained Mreg-bc cells on day 7; Iso_{Mreg-bc} is the signal of isotype control-stained Mreg-bc cells on day 7; Syndecan-3_{flask} is the specific signal of Syndecan-3-stained flask-cultured Mreg cells on day 7; Iso_{flask} is the signal of isotype control-stained flask-cultured Mreg cells on day 7. The relative expression of Syndecan-3 by Mregs-bc cells and flask-cultured Mregs can be conveniently determined by using standard flow cytometry methods. According to a preferred embodiment, the relative expression of Syndecan-3 by the Mreg-bc cell compared to flask-cultured Mregs (expressed as % expression) is less than 50%, more preferably less than 40%, less than 30%, less than 20%, less than 15%, less than 10%, less than 5%, or less than 1%.

Preferably, the Mreg-bc cells of the invention expresses at least one of the markers CD85h and CD258, more preferably both markers. The Mreg-bc cell preferably expresses one or more of the markers Clec-9a, CD103 and CD10. Stated differently, the invention provides an Mreg-bc cell that expresses one or more of the markers Clec-9, CD103 and CD10. Preferably, said cell expresses at least one of the markers CD85h and CD258, more preferably both markers. The Mreg-bc cell preferably does furthermore not express one or more of the markers CD38, CD209 and Syndecan-3 (or expresses one or more of these markers only at a comparatively low level). In a particularly preferred embodiment, the Mreg-bc cell provided herein does not express the markers CD38, CD209 and Syndecan-3, and at the same time expresses the markers CD85h, CD258, Clec-9, CD103 and CD10. Hence, the Mreg-bc cells provided by the method of the invention are macrophages which can be described by one of the following marker patterns:
(1). CD45⁺, CD85h⁺, CD38^{-/low};
(2). CD45⁺, CD85h⁺, CD209^{-/low};
(3). CD45+, CD85h+, Syndecan 3^{-/low};
(4). CD45⁺, CD258⁺, CD38^{-/low};
(5). CD45⁺, CD258⁺, CD209^{-/low};
(6). CD45+, CD258⁺, Syndecan 3^{-/low};
(7). CD45⁺, CD85h⁺, CD258⁺, CD38^{-/low};
(8). CD45⁺, CD85h⁺, CD258⁺, CD209^{-/low};
(9). CD45+, CD85h+, CD258⁺, Syndecan 3^{-/low};
(10). CD45⁺, CD85h⁺, CD258⁺, CD38^{-/low}, CD209^{-/low};
(11). CD45⁺, CD85h⁺, CD258⁺, CD38^{-/low}, Syndecan 3^{-/low};
(12). CD45⁺, CD85h⁺, CD258⁺, CD209^{-/low}, Syndecan 3^{-/low};
(13). CD45⁺, CD85h⁺, CD258⁺, CD38^{-/low}, CD209^{-/low}, Syndecan 3^{-/low};
(14). CD33⁺, CD85h⁺, CD38^{-/low};
(15). CD33⁺, CD85h⁺, CD209^{-/low};
(16). CD33+, CD85h+, Syndecan 3^{-/low};
(17). CD33⁺, CD258⁺, CD38^{-/low};
(18). CD33⁺, CD258⁺, CD209^{-/low};
(19). CD33+, CD258⁺, Syndecan 3^{-/low};
(20). CD33⁺, CD85h⁺, CD258⁺, CD38^{-/low};
(21). CD33⁺, CD85h⁺, CD258⁺, CD209^{-/low};
(22). CD33+, CD85h+, CD258⁺, Syndecan 3^{-/low};
(23). CD33⁺, CD85h⁺, CD258⁺, CD38^{-/low}, CD209^{-/low};
(24). CD33⁺, CD85h⁺, CD258⁺, CD38^{-/low}, Syndecan 3^{-/low};
(25). CD33⁺, CD85h⁺, CD258⁺, CD209^{-/low}, Syndecan 3^{-/low};
(26). CD33⁺, CD85h⁺, CD258⁺, CD38^{-/low}, CD209^{-/low}, Syndecan 3^{-/low};
(27). CD11b⁺, CD85h⁺, CD38^{-/low};
(28). CD11b⁺, CD85h⁺, CD209^{-/low};
(29). CD11b+, CD85h+, Syndecan 3^{-/low};
(30). CD11b⁺, CD258⁺, CD38^{-/low};
(31). CD11b⁺, CD258⁺, CD209^{-/low};
(32). CD11b+, CD258⁺, Syndecan 3^{-/low};
(33). CD11b⁺, CD85h⁺, CD258⁺, CD38^{-/low};
(34). CD11b⁺, CD85h⁺, CD258⁺, CD209^{-/low};
(35). CD11b⁺, CD85h+, CD258⁺, Syndecan 3^{-/low};
(36). CD11b⁺, CD85h⁺, CD258⁺, CD38^{-/low}, CD209^{-/low};
(37). CD11b⁺, CD85h⁺, CD258⁺, CD38^{-/low}, Syndecan 3^{-/low};
(38). CD11b⁺, CD85h⁺, CD258⁺, CD209^{-/low}, Syndecan 3^{-/low};
(39). CD11b⁺, CD85h⁺, CD258⁺, CD38^{-/low}, CD209^{-/low}, Syndecan 3^{-/low};
(40). CD45⁺, CD11b⁺, CD85h⁺, CD38^{-/low};
(41). CD45⁺, CD11b⁺, CD85h⁺, CD209^{-/low};
(42). CD45⁺, CD11b+, CD85h+, Syndecan 3^{-/low};
(43). CD45⁺, CD11b⁺, CD258⁺, CD38^{-/low};
(44). CD45⁺, CD11b⁺, CD258⁺, CD209^{-/low};
(45). CD45⁺, CD11b⁺, CD258⁺, Syndecan 3^{-/low};
(46). CD45⁺, CD11b⁺, CD85h⁺, CD258⁺, CD38^{-/low};
(47). CD45⁺, CD11b⁺, CD85h⁺, CD258⁺, CD209^{-/low};
(48). CD45⁺, CD11b⁺, CD85h+, CD258⁺, Syndecan 3^{-/low};
(49). CD45⁺, CD11b⁺, CD85h⁺, CD258⁺, CD38^{-/low}, CD209^{-/low};
(50). CD45⁺, CD11b⁺, CD85h⁺, CD258⁺, CD38^{-/low}, Syndecan 3^{-/low};
(51). CD45⁺, CD11b⁺, CD85h⁺, CD258⁺, CD209^{-/low}, Syndecan 3^{-/low};
(52). CD45⁺ CD11b⁺ CD85h⁺ CD258⁺ CD209^{-/low} Clec-9a⁺
(53). CD45⁺, CD11b⁺, CD85h⁺, CD258⁺, CD38^{-/low}, CD209^{-/low}, Syndecan 3^{-/low};

It is particularly preferred that the Mreg-bc cell does not or not to a significant extent express CD34. CD34 is a commonly used marker for hematopoietic stem cells and progenitor cells in clinical hematology. It is preferred that less than 30% of the Mreg-bc cells obtained by the method of the invention express CD34 after 7 days of culturing, more preferably less than 20%, less than 15%, less than 10%, less than 5%, or less than 1%. In one embodiment of the invention, the macrophage cells of the invention are derived from a human subject, i.e. are of human origin.

The marker profile of the Mreg-bc cell can conveniently be determined by using standard flow cytometry methods. Methods and reagent that are useful for determining the surface markers of cells have been extensively described in the literature. Preferably, the marker phenotype of the Mreg-bc cells of the invention is determined as described in the Example part.

It was found herein that the transition of monocytes to regulatory macrophages occurs gradually. During the generation of cells, the initial population of CD14+ monocytes undergoes a gradual loss of CD14 expression at the cell surface. Therefore, in another preferred embodiment the differentiated Mreg-bc cell obtained from the method of the invention does not or not to a significant extent express the marker CD14 which is characteristic for the monocytic lineage. Down-regulation of CD14 during Mreg-bc development can be expressed as the percentage of CD14 expression on Mregs-bc at day 7 compared to monocytes on day 0 of culture. The expression of CD14 is proportional to the difference in fluorescence intensity between an isotype control-stained cell and the specific CD14 signal. Hence, % down-regulation = 100 - 100 × (CD14_{d7} - Iso_{d7})/(CD14_{d0} - Iso_{d0}) where: CD14_{d7} is the specific signal on day 7; Iso_{d7} is the isotype control signal on day 7; CD14_{d0} is the specific signal on day 0; Iso_{d0} is the isotype control signal on day 0. The down-regulation of CD14 by Mregs-bc cells can be conveniently determined by using standard flow cytometry methods. It is preferred that during the process of monocyte to Mreg-bc differentiation, the expression of CD14 is down-regulated by more than 25%, preferably more than 50%, 60%, 70%, 80%, 90%, and more preferably by more than 95%.

The Mreg-bc cells are particularly suitable for being used for therapeutic purposes, as explained in more detail below. In concept, Mreg-bc therapy is a gain-of-function therapy meaning that administration of Mreg-bc cells with immunosuppressive, anti-inflammatory or tissue-reparative functions will complement a deficiency of those cellular functions in the recipient. By applying suitably large doses, it will be possible to restore or exceed said activities in the recipient. In transplant and autoimmune models, Mreg-bc treatment has a therapeutic effect that persists beyond their own lifespan in the recipient. This enduring effect can be explained by the impact of Mreg-bc treatment upon the recipient T cells. Administration of Mreg-bc cells may influence recipient T cell responses in three complementary ways.
(a) Mreg-bc cells directly interact with recipient T cells which results in specific T cell deletion or conversion into activated induced regulatory T cells (iTregs).
(b) Mreg-bc cells alter the behaviour of recipient dendritic cells through direct interaction or release of anti-inflammatory mediators. One important function of Mreg-bc cells may be to die in a suitably self-conditioned environment and give-up antigens to recipient dendritic cells which in turn specifically suppress recipient T cells.
(c) Mreg-bc cells exert active or passive non-specific suppressive through the release of soluble mediators that may act directly or exert effects through recipient myelomonocytic cells.

In addition to their T cell-suppressive activity, the Mreg-bc cells of the invention exhibit additional characteristic features that make them valuable for therapeutic use. As shown in Example 6, the Mreg-bc cells of the invention secrete biologically relevant amounts of Vascular Endothelial Growth Factor (VEGF-A) and other pro-angiogenic mediators upon stimulation with toll-like receptor (TLR) ligands, such as lipolysaccharide (LPS), monophosphoryl lipid A (MPLA) or High Mobility Group Box protein 1 (HMGB1). As a consequence, the Mreg-bc cells of the invention are suitable for treating diseases and conditions where the induction of angiogenesis is desired, such as in ischaemic diseases and conditions.

In a fourth aspect, the invention refers to a pharmaceutical composition comprising the Mreg-bc cell of the second aspect of the invention. The pharmaceutical composition will comprise, as a first component, an effective amount of the Mreg-bc cells of the invention. As used herein, an effective amount of the Mreg-bc cells to be administered to the patient will be in the range of about 1×10⁴ to about 1×10⁸/kg body weight, preferably between about 1×10⁵ and about 1×10⁷/kg body weight, and more preferably between about 1×10⁶ and about 9×10⁶/kg body weight, such as about 1×10⁶/kg, about 2×10⁶/kg, about 3×10⁶/kg, about 4×10⁶/kg, about 5×10⁶/kg, about 6×10⁶/kg, about 7×10⁶/kg, or about 8×10⁶/kg body weight of the patient to be treated.

Apart from the cells, the pharmaceutical composition can comprise further excipients, such as buffers, pH regulating agents, preservatives, and the like. The nature and amounts of the excipients included in the pharmaceutical composition of the invention will depend on the intended route of administration. Generally, different routes of administration are feasible for providing the Mreg-bc cells of the invention to a patient in need of treatment. Preferably, the pharmaceutical composition of the invention will be formulated for parenteral administration, such as subcutaneous, intramuscular, intravenous or intradermal administration. It is particularly preferred that the Mreg-bc cells or a composition comprising said cells or fractions are administered to the patient by intravenous administration.

The formulation of the Mreg-bc cells of the invention into pharmaceutical compositions can be achieved by applying routine methods known in the field of drug formulation. Suitable methods are described, for example, in standard textbooks. Pharmaceutical compositions suitable for intravenous administration by injection or infusion normally include sterile aqueous solutions or suspensions and sterile powders for the extemporaneous preparation of sterile solutions or suspensions. The composition intended for injection must be sterile and should be fluid in order to allow a convenient handling in a syringe or infusion bag.

The composition should be stable under the conditions of administration and is preferably preserved against the contaminating action of microorganisms such as bacteria and fungi, for example, by including parabens, chlorobutanol, phenol, ascorbic acid, thimerosal, and the like into the composition. For intravenous administration, suitable carriers may comprise physiological saline, bacteriostatic water, Cremophor EL^{™} (BASF) or phosphate buffered saline (PBS). The carrier may also be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Sterile injectable solutions can be prepared by incorporating the cells in the required amount in an appropriate solvent with one or more of the above mentioned ingredients followed by sterile filtration. Generally, suspensions are prepared by incorporating the active compound, i.e. the cells, into a sterile vehicle that contains a basic dispersion medium and the required other ingredients from those mentioned above. In case of sterile powders for the preparation of sterile injectable solutions, methods of preparation are vacuum drying and freeze-drying that yields a powder of the cells plus any additional desired ingredient from a previously sterile-filtered solution thereof.

The composition intended for infusion or injection will have a volume of between 50 and 500 ml, wherein a volume of between 90 ml and 250 ml is particularly preferred, and wherein a volume of between 90 ml and 150 ml is even more preferred.

The Mreg-bc cells can be administered to a patient in need of treatment by different administration regimens. For example, where the cells or cell fractions are administered to the patient by intravenous infusion, the total amount of Mreg-bc cells or Mreg-bc cell fractions to be administered can be supplied by one or more than one infusion. In a preferred embodiment, the Mreg-bc cells or cell fractions are supplied to the patient through an infusion set with a 200 µm filter. The suspension comprising the Mreg-bc cells or Mreg-bc cell fractions may be primed with 0.9% NaCl. The suspension may be given in a single infusion, more preferably a short-term infusion within less than 60 min, e.g. within 60 min, 30 min, 20 min or 15 min. Preferably, a central venous catheter is used for administering the Mreg-bc suspension.

The administration of the Mreg-bc cells or cell fractions can be accompanied by the preceding, simultaneous or subsequent administration of other active agents. For example, where the Mreg-bc cells or cell fractions of the invention are administered to prevent an immune response in a patient receiving an organ transplant, an immunosuppressive drug may be administered together with the cells or cell fractions of the invention. Immunosuppressive drugs that are routinely used in the field of transplantation medicine comprise, but are not limited to, cyclosporin A (CSA), tacrolimus, azathioprine (AZA), mycophenolate mofetil, rapamycin, and steroids (STE). Generally, the presence of immunosuppressive drugs in the recipient blood does not affect the effectiveness of the cells or cell fractions of the invention.

Although the Mreg-bc cells obtained from the method described in the first aspect of the invention exhibit a stable phenotype, it is recommended for safety reasons that the Mreg-bc cells are administered within 24 hours after harvesting them from the cell cultures. Preferably, the cells are administered within 20 hours, within 16 hours, within 12 hours, within 8 hours, or within 4 hours after harvesting the cells from the cultures.

In a fourth aspect, the invention refers to the therapeutic application of Mreg-bc cells according to the second aspect of the invention or compositions according to the third aspect of the invention. As indicated elsewhere herein, the Mreg-bc cells provided by the present invention exhibit a number of pharmacological properties, such as immunosuppressive, immunoregulatory, angiogenic and anti-inflammatory properties, which make them highly suitable for being used in immunosuppressive, anti-inflammatory or tissue-reparative therapies. For example, the artificially induced Mreg-bc cells of the invention are T cell-suppressive and mediate an active deletion of activated T cells. As such, the cells are highly suitable for use as an adjunct immunosuppressive therapy in a variety of immunologically-mediated diseases, such as organ transplantation.

Accordingly, in one embodiment of the invention, an Mreg-bc cell according to the second aspect of the invention or a pharmaceutical composition according to the third aspect of the invention is used in a method of suppressing transplant rejection and/or prolonging transplant survival in a subject receiving a transplant. The invention thus refers to a method of suppressing transplant rejection and/or prolonging transplant survival in a subject receiving a transplant, comprising (i) the administration of an effective amount of an Mreg-bc cell according to the second aspect of the invention, or (ii) the administration of a pharmaceutical composition according to the third aspect of the invention. Preferably, the transplant is an organ, tissue or cell transplant. The type of organ to be transplanted is not limited according to the invention, but it will preferably be a kidney, liver, heart, lung, or pancreas. It is particularly preferred that the organ to be transplanted to the recipient is a human organ.

The Mreg-bc of the present invention can also be used for suppressing transplant rejection and/or prolonging transplant survival in cases where the transplant is a tissue transplant rather than an organ transplant. Again, the tissue to be transplanted into the recipient is not particularly limited. The rejection of any tissue that was derived from an allogeneic donor in the recipient can be prevented or ameliorated by the Mreg-bc of the present invention. The tissue to be transplanted will preferably be a human tissue, such as an intestinal, cornea, skin, composite tissue, bone marrow, or pancreatic islet tissue.

The Mreg-bc prepared according to the method of the invention can also support cell transplant into a recipient by suppression of the immune response in the recipient. Where the transplant is a cell transplant, the nature of the cell to be transplanted is generally not limited, but it is preferred that the cell to be transplanted is selected from the group consisting of an adult stem cell transplant, an isolated hepatocyte transplant or a leukocyte cell transplant. The Mreg-bc cells of the invention are also capable of producing soluble factors that promote the homing and engraftment of adult stem cells, such as cathelicidin. In a preferred embodiment of this invention, Mreg-bc cells are used to facilitate engraftment of haematopoietic stem cells (HSC) after bone marrow or HSC transplantation.

To suppress transplant rejection in the recipient and induce acceptance of an allogeneic organ, tissue or cell transplant, the Mreg-bc cells of the invention or a pharmaceutical composition comprising the Mreg-bc cells can be administered intravenously by injection or infusion, as described above. The injection or infusion can be given either pre-operatively or post-operatively. If the Mreg-bc cells are administered pre-operatively, they will be administered to the recipient at least one time, preferably two times, and more preferably three times before the operation. It is preferred that the Mreg-bc are administered to the recipient not earlier than one week before operation, e.g. 6 days, 5 days, 4 days, 3 days, 2 days, or 1 day before operation. If the Mreg-bc cells are administered post-operatively, a first administration will be given preferably within 24 hours after operation, more preferably within 36 hours, 48 hours, 60 hours, or 72 hours after operation. Alternatively, in stably immunosuppressed transplant recipients, Mreg-bc therapy may be administered at any time after transplantation. Alternatively, Mreg-bc may be administered to transplant recipients undergoing acute or chronic transplant rejection. The Mregs-bc are then capable of repelling the T-cell response of the recipient's immune system against the transplant and to persist in the recipient's body (especially spleen, liver, lungs and bone marrow) for a sufficiently long period of time to confer long-term transplant acceptance to the recipient.

When using the Mreg-bc of the invention for suppressing transplant rejection or prolonging transplant survival in a subject receiving a transplant, the transplant will normally be an allogeneic transplant, i.e. a transplant which originates from a donor that, although genetically different, belongs to the same species as the recipient. In this case, the Mreg-bc cells are generated based on blood monocytes that were obtained from said donor. The monocytes can be obtained from a living donor or a dead donor. In case of a dead donor, i.e. a body donation, the body of the donor is normally flushed with a perfusion medium by canalisation of the principal artery for the purpose of organ preservation. The venous blood is removed from the body and can be collected for preparing the Mreg-bc according to the method described herein. Alternatively, Mreg-bc can also be prepared from myelomononuclear cells that are isolated from the donor's spleen. In the case of post-operative application of the Mreg-bc that were prepared from a deceased donor, a rejection of the transplanted organ can be prevented by the administration of immunosuppressants which are routinely used for this purpose during organ transplantation.

In another embodiment, the Mreg-bc cells according to the second aspect of the invention or a pharmaceutical composition according to the third aspect of the invention is used in a method of promoting or sustaining the engraftment or effect of regulatory T cell cell-based medicinal products. The invention thus also refers to a method of promoting or sustaining the engraftment or effect of regulatory T cell cell-based medicinal products in a subject comprising (i) the administration of an effective amount of an Mreg-bc cell according to the second aspect of the invention, or (ii) the administration of a pharmaceutical composition according to the third aspect of the invention.

Apart from the immunoregulatory and immunosuppressive properties, the Mreg-bc cells of the invention have anti-inflammatory properties which allow for the abrogation of chronic inflammatory immune processes. Accordingly, the Mreg-bc cells provided herein are also useful for treating diseases or disorders that are characterized by a deregulated immune status or an excessive inflammatory reaction, in particular chronic inflammatory diseases. Such diseases or disorders include, for example, autoimmune diseases, inflammatory diseases and hypersensitivity reactions.

Thus, in yet another embodiment, the Mreg-bc cell according to the second aspect of the invention or pharmaceutical composition according to the third aspect of the invention is used in a method of treating or preventing an autoimmune disease, an inflammatory disease, or a hypersensitivity reaction.

Where the Mreg-bc are used for treating an autoimmune disease, said disease may be (a) principally T cell-mediated, (b) principally antibody-mediated or (c) principally mediated by other cellular components of the immune system. The disease may be a local or systemic autoimmune condition. The type of autoimmune conditions to be treated with the Mreg-bc therapy is not limited according to the invention, and includes systemic lupus erythematosus (SLE), scleroderma, Sjögren's syndrome, polymyositis, dermatomyositis, and other systemic autoimmune conditions; rheumatoid arthritis (RA), juvenile rheumatoid arthritis, and other inflammatory arthritides; ulcerative colitis, Crohn's disease, and other inflammatory bowel diseases; autoimmune hepatitis, primary biliary cirrhosis, and other autoimmune liver diseases; cutaneous small-vessel vasculitis, granulomatosis with polyangiitis, eosinophilic granulomatosis with polyangiitis, Behçet's disease, thromboangiitis obliterans, Kawasaki disease, and other large-, medium- or small-vessel vasculitides of autoimmune aetiology; Multiple sclerosis (MS) and neuroimmunological disorders; Type I diabetes, autoimmune thyroid dysfunction, autoimmune pituitary dysfunction, and other autoimmune endocrinological disorders; haemolytic anaemia, thrombocytopaenic purpura and other autoimmune disorders of the blood and bone marrow; psoriasis, pemphigus vulgaris, pemphigoid and other autoimmune dermatological conditions.

The Mreg-bc cells are also effective for treating acute or chronic inflammatory diseases, and diseases with a pathophysiologically significant inflammatory component. The inflammatory disease to be treated may be local or systemic. The type of inflammatory diseases or condition that benefit from treatment with Mreg-bc is not limited and includes, but is not limited to arterial occlusive diseases, such as peripheral artery occlusive disease (pAOD), critical limb ischaemia, arteriosclerosis, cerebral infarction, myocardial infarction, renal infarction, intestinal infarction, angina pectoris, and other conditions caused by arterial occlusion or constriction; microvascular angina, also known as cardiac syndrome X; inflammation associated systemic with metabolic disorders, including Type II diabetes and obesity-related metabolic syndrome; dermatological diseases, including eczema. Preferably, the inflammatory disease to be treated is characterized by chronic inflammation of the intima of an arterial wall, e.g. myocardial infarction, stroke, critical limb ischemia vasculitis and pAOD.

Where the treatment of a hypersensitivity reaction is desired, the hypersensitivity reaction is preferably selected from the group of asthma, eczema, allergic rhinitis, angioedema, drug hypersensitivity, and mastocytosis.

The treatment of pAOD is particularly preferred. It is known that pAOD in patients who are unfit for revascularisation procedures, owing either to the extent or location of their arterial occlusions, or to significant co-morbidities, is a severely debilitating and prevalent condition for which amputation is the only therapeutic option. Amputation remains a treatment of last resort and is associated with relatively high mortality, and only a minority of patients subsequently recover full mobility. In the course of the present invention, it was found that the Mreg-bc cells obtained from the method described herein have angiogenic properties. The Mreg-bc actively promote neovascularisation, i.e. the formation of new blood vessels, through the basal and stimulated expression of pro-angiogenic growth factors, such as VEGF, FIGF (VEGF-D), PDGFB and MDK. In particular, Mreg-bc cells produce high levels of Vascular Endothelial Growth Factor (VEGF) upon stimulation with TLR4 ligands. In particular, it was found herein that Mreg-bc induce the expression of the vascular endothelial growth factor C (VEGF-C). It has been reported in the literature that VEGF-C is an angiogenic factor that effectively stimulates neovascularisation in vivo [16].

In one embodiment, Mreg-bc cells are injected intramuscularly or subcutaneously into an ischaemic limb. In the ischaemic tissue, Mreg-bc cells will inevitably be exposed to microbial components and necrotic tissue components (e.g. HMGB1) that act as TLR4 agonists. Hence, Mreg-bc can be used to promote tissue regeneration through local secretion of pro-angiogenic growth factors. In another embodiment, Mreg-bc cells may be stimulated ex vivo with TLR ligands during the manufacturing process to ensure their high-level production of VEGF. Said TLR ligands may include, but are not limited to, lipopolysaccharide (LPS) or monophosphoryl lipid A (MPLA). The pAOD to be treated with the Mreg-bc of the invention may be a pAOD of any grade or category. For example, the pAOD may be a pAOD of grade I, categories 1-4, or grade II-IV pAOD.

As the Mreg-bc of the invention have angiogenic properties, their use in other diseases or conditions that require neovascularisation is contemplated herein. Hence, the invention also relates to the Mreg-bc cell according to the second aspect of the invention or pharmaceutical composition according to the third aspect of the invention which is used in a method of inducing angiogenesis or vasculogenesis in hypoxic tissues, promoting tissue-repair processes by participating in tissue remodelling, tissue regeneration, preventing or reducing fibrosis, reducing ischemic pain or avoiding major limb amputation. The invention thus refers to a method of inducing angiogenesis or vasculogenesis in hypoxic tissues, promoting tissue-repair processes by participating in tissue remodelling, tissue regeneration, preventing or reducing fibrosis, reducing ischemic pain or avoiding major limb amputation comprising (i) the administration of an effective amount of an Mreg-bc cell according to the second aspect of the invention, or (ii) the administration of a pharmaceutical composition according to the third aspect of the invention.

The treatment of autoimmune diseases, inflammatory diseases, or hypersensitivity reactions can be achieved either with Mreg-bc which are derived from monocytes which are allogeneic to the patient, as described above in the context with transplantation applications, or with monocytes which are autologous to the patient in need of treatment. Where possible, the treatment of autoimmune diseases, inflammatory diseases, or hypersensitivity reactions will be performed with autologous monocytes. For this purpose, the Mregs-bc can be administered intravenously with or without simultaneous local intramuscular injections.

In yet another embodiment, the Mreg-bc cell according to the second aspect of the invention or pharmaceutical composition according to the third aspect of the invention is used as a vehicle to deliver gene therapy. The invention thus refers to a method of delivering gene therapy comprising (i) the administration of an effective amount of an Mreg-bc cell according to the second aspect of the invention which comprises a transgene, or (ii) the administration of a pharmaceutical composition comprising an Mreg-bc cell according to the second aspect of the invention which comprises a transgene.

According to a fifth aspect, the invention refers to a process for preparing an immunoregulatory T cell, said process comprising:
(a) obtaining T cells of a subject;
(b) co-culturing the T cells with an immunoregulatory macrophage cell as described above;
(c) obtaining the immunoregulatory T cells from the culture medium.

As described elsewhere herein, T cells that have been co-cultured with Mregs-bc inhibit T cell proliferation. Accordingly, the immunoregulatory T cells obtained from the above methods can be used, either alone or in combination with the Mreg-bc cells of the invention, for the treatment of any of the diseases or disorders discussed elsewhere herein. In a first step, T cells from a blood sample of a subject are obtained. The cells can be obtained, e.g. from a blood sample or aphere-sate, or from the subject's tissue, e.g. from bone marrow or the spleen. The cells can be obtained by conventional methods, e.g. in case of cells from the blood by venepuncture. The T cells used in the above method will be, for example, CD3+ T cells or subsets thereof. Before being co-cultured with the Mreg-be cells, the CD3+ T cells can be purified or enriched by conventional methods, e.g. by magnetic microbead separation or flow cytometry sorting.

The T cells are then contacted with Mregs-bc of the present invention. The cells can be contacted in different Mreg:Treg ratios. For example, the cell fractions can be contacted in a Mreg:Treg ratio of between 1:5 to 5:1, preferably between 1:2 to 2:1. More preferably, Mreg:Treg ratio is about 1:1. Different media can be used for the co-culturing method. The media can be those described above in connection with the method for preparing Mreg-bc cells. In a preferred embodiment the medium is X-vivo 10 from Lonza. The medium may contain further additives such as M-CSF and/or GM-CSF, preferably human recombinant M-CSF and/or GM-CSF. The amount of M-CSF and/or GM-CSF will be in the range mentioned elsewhere herein, e.g. 5-100 ng/ml, preferably 20-25 ng/ml. The medium may also contain other additives, such as Glutamax in an amount of 1-5 mM, preferably 2 mM.

The cells will be co-cultured for 1-8 days, preferably for at least 3 days, at least 4 days, or at least 5 days. After the pre-determined culturing period, the T cells can be re-isolated by conventional methods, e.g. by enriching the cells for CD4⁺ CD25⁺ TIGIT⁺ FoxP3⁺ Tregs. If necessary, the cells can be further formulated to pharmaceutical products.

Also disclosed herein is a method for detecting immunoregulatory macrophage cells, comprising
(a) providing a sample that comprises macrophage cells;
(b) detecting in said sample the presence of the DHRS9 protein and/or the expression of the DHRS9 gene;
wherein the presence of the DHRS9 protein and/or the expression of the DHRS9 gene indicates that the sample comprises immunoregulatory macrophage cells.

The method can be used for discriminating between immunoregulatory macrophage cells (Mregs) and macrophages in other activation states, such as monocyte-derived macrophages (Mφ), including resting Mφ, LPS + IFNγ-stimulated Mφ, IL-4-stimulated Mφ and immunoglobulin (Ig)-stimulated Mφ. Since the expression of DHRS9 was found only in Mregs, this marker can be used for identifying Mregs in a heterogeneous population of macrophages, e.g. a population comprising Mregs and at least one of the following macrophage types: resting Mφ, LPS + IFNγ-stimulated Mφ, IL-4-stimulated Mφ, and immunoglobulin (Ig)-stimulated Mφ. Detection of the DHRS9 marker can be achieved by flow cytometry using standard antibodies against the DHRS9 polypeptide or fragments thereof. Expression of DHRS9 can be detected by PCR, RT-PCR, real-time PCR, and other routine methods.

Also disclosed herein is a method for isolating immunoregulatory macrophage cells from a heterogeneous population of macrophages, comprising
(a) providing a heterogeneous population of macrophages;
(b) isolating immunoregulatory macrophage cells via their affinity to molecules that specifically bind to the DHRS9 protein.

For example, in one embodiment, the immunoregulatory macrophage cells within the heterogeneous population of macrophages can be isolated by binding to antibodies directed against DHRS9. Such antibodies can be of monoclonal or polyclonal origin. In a preferred embodiment, the anti-DHRS9 antibodies can be immobilized to a solid phase, such as the bottom of a microtiter plate well. The macrophage population is incubated in the well to allow binding of the anti-DHRS9 antibodies to the DHRS9 which is present on the surface of the immunoregulatory macrophage cells. After washing away unbound macrophages, a homogenous population of immunoregulatory macrophage cells is obtained. In another embodiment, the anti-DHRS9 antibodies can be immobilized on the surface of magnetic beads. The beads are incubated with the macrophage population to allow binding of the antibodies to DHRS9. After separating the beads from the solution containing the macrophage population, a homogenous population of immunoregulatory macrophage cells is obtained.

Accordingly, also disclosed herein is the use of a molecule that specifically binds to DHRS9, in particular an anti-DHRS9 antibody, for the detection or isolation of immunoregulatory macrophage cells.

### EXAMPLES

The Mreg-bc cells were manufactured in accordance with current GMP principles for the production of sterile medicinal products. Attention is paid at every processing step that products, materials and equipment are protected against contamination and impurities.

### Example 1: Preparation of Mregs-be cells

Healthy human donors were subjected to leukapheresis to collect peripheral blood mononuclear cells (PBMC) which are used as starting material for Mreg-bc generation. All donors were screened for relevant disease markers, including infectious diseases, not more than 30 days prior to leukapheresis. Donors were re-screened for the same disease markers on the day of leukapheresis. Leukapheresis was performed using the Terumo BCT Cobe Spectra device or equivalent.

CD14+ monocytes were isolated from the leukapheresis product using the Miltenyi CliniMACS^{®} system in accordance with the manufacturer's instructions. Briefly, the leukapheresis product was transferred into a bag which was filled with PBS/EDTA-buffer containing 0.5% human serum albumin (HSA). The cells were washed once before labelling with CliniMACS^{®} CD14 reagent according to the manufacturer's instructions. The labelled cell suspension was connected to a sterile tubing set and installed on the CliniMACS^{®} device in order to isolate CD14+ monocytes by magnetic separation. The positively-isolated CD14+ monocyte fraction was washed with culture medium to remove the CliniMACS^{®} separation buffer.

Monocyte density was then adjusted to 10⁶ cells/ml in cell culture medium. CD14+ monocytes were taken for analysis by flow cytometry as an in-process control. Cell numbers for process-related calculations were determined using an automated blood counter using the WBC parameter as the total leukocyte number. The viability of all cell types was assessed by flow cytometry.

The isolated CD14+ monocytes were re-suspended at a density of 10⁶ cells/ml in RPMI medium that had been supplemented with 10% male-only human AB serum (pooled and heat-inactivated), 2 mM GlutaMAX^{™} and 25 ng/ml recombinant human monocyte colony-stimulating factor (M-CSF).

This monocyte suspension was distributed into Miltenyi^{®} cell differentiation bags, such that each bag was seeded with 1 × 10⁶ cells/cm² internal surface area. For cultivation, the differentiation bags were laid flat on shelves within an incubator which was set to 36-38°C, 5 ± 1% CO₂, ≥ 60% humidity. The monocytes were allowed to precipitate onto the lower leaf of the culture bags over the course of 1 day. On day 1, the bags were inverted to allow monocytes to adhere to the opposite leaf. The cultures were maintained in the incubator for a further 5 days.

To induce the final differentiation of monocytes into Mregs-bc and to induce indoleamine 2,3-dioxygenase (IDO) expression, monocytes were stimulated by the addition of 25 ng/ml IFN-γ. After the addition of IFN-y, the differentiation bags were inverted another time. The bags were then incubated for a further 18-24 h at 36-38°C, 5 ± 1% CO₂, ≥ 60% humidity.

On day 7, the differentiated Mregs-bc were harvested. Cells from all parallel culture bags were pooled and washed prior to phenotypic and functional analyses.

### Example 2: Phenotypic characterization of Mregs-be

The phenotype of Mregs-be obtained from the method in Example 1 was analyzed in detail. In culture, the macrophages exhibit a distinctive morphology with the cells adopting a tessellating, epithelioid morphology to form almost confluent monolayers (see Figure 6A). Individual macrophages are large, densely granular cells with a prominent central body and a thin cytoplasmic skirt, which spreads symmetrically over the surface of the culture vessel. Ultrastructural examination of macrophages by transmission electron microscopy confirms the impression of a large, flattened cell adhering very closely to the underlying surface (see Figure 6B). In most respects, the ultrastructural appearance of the macrophages is typical of an activated macrophage: processes extend from the outer perimeter and upper surface of the cells; the nuclei appear active with abundant fine chromatin; and, the cytoplasm contains numerous endocytic vesicles, lipid inclusions and a prominent smooth endoplasmic reticulum.

The cell-surface phenotype of Mreg-bc cells was characterised by flow cytometry. To prepare Mreg-be cells for analysis by flow cytometry, the cells were harvested and washed once in Ca²⁺/Mg²⁺ free DPBS before being resuspended at 1-5 × 10⁵ cells/100 µl in Ca²⁺/Mg²⁺ free DPBS containing 1% BSA, 0.02% NaN₃, and 10% FcR block (Miltenyi). Samples were then incubated at 4°C for 15 min. Fluorochrome-conjugated antibodies were obtained from different manufacturers and applied to the cell suspensions and samples were vortexed before incubation at 4°C for 20 min in the dark. After addition of 10 µl 7-AAD, each sample was briefly vortexed and incubated for a further 10 min at 4°C in the dark. Samples were subsequently washed twice in cold Ca²⁺/Mg²⁺ free DPBS and resuspended for analysis. FASER reagents (Miltenyi) used in 2 rounds according to the manufacturer's instructions were used to enhance Clec-9a signals. For intracellular staining, cells were first stained for cell surface antigens as described above, then fixed and permeabilized using an Intracellular Fixation & Permeabilization Buffer Set (eBioscience) according to the manufacturer's instructions. Cells were resuspended in permeabilization buffer containing 10% FcR block and were then incubated for 15 min at 4°C in the dark. Fluorochrome conjugated antibodies were applied to the cell suspensions and samples were briefly vortexed before incubation at 4°C for 30 min in the dark. Cells were washed twice in Permeabilization Buffer and resuspended for analysis. Data were acquired on a Canto II flow cytometer (BD Biosciences, Germany) and analyzed with FlowJo 7.6 software (TreeStar, USA) or Kaluza 1.1 software (Beckman Coulter, Germany).

This flow cytometry analysis reveals that viable human Mreg-bc cells of the invention exhibit a CD14^{-/low} CD209^{-/low} CD16^{-/low} CD80^{-/low} CD86⁺ CD10^{+/-} CD103^{+/-} CD38^{-/low} CD85h⁺ CD258⁺ Syndecan-3^{-/low} Clec-9a⁺ phenotype (Fig. 2).

### Example 3: Uniqueness of the Mreg phenotype

The phenotypic relationship of Mregs to macrophages in other states of activation known in the prior art was established by generating a panel of macrophage types for comparison with Mregs in terms of morphology, cell-surface marker expression, cytokine production, and global gene expression profiles. Mregs could be readily distinguished from all these other macrophage types by their characteristic morphology (see Figure 6C) and by their distinct cell surface phenotype (see Figure 6D). In particular, Mregs were found to be unique in downregulating CD14 and in their lack of cell-surface CD16, TLR2 and CD163 expression.

Mregs and the panel of comparator macrophages were also distinguished by their cytokine and chemokine production profiles. Mregs constitutively produce only small amounts of TNF-α and IL-6, and do not secrete detectable amounts of IL-12p40. Mregs express detectable levels of TGF-β and high amounts of IL-1Ra, but notably less IL-10 than other macrophage types. This cytokine secretion profile remains relatively stable after exposure to IFN-γ and LPS.

To identify markers exclusively expressed by Mregs, Mregs and IFN-γ-Mφ were generated according to previously described methods [10] from peripheral blood leucocytes obtained as a byproduct of thrombocyte collection from healthy donors. Briefly, CD14+ monocytes were isolated from Ficoll-prepared PBMC by positive-selection with anti-CD14 microbeads (Miltenyi, Bergisch-Gladbach) and were then plated in 6-well Cell+ plates (Sarstedt, Nümbrecht) at 10⁵ cells/cm² in RPMI-1640 (Lonza, Cologne) supplemented with 10% heat-inactivated human AB serum (Lonza), 2 mM Glutamax (Invitrogen, Karlsruhe), 100 U/mL penicillin (Lonza), 100 µg/mL streptomycin (Lonza), and rhM-CSF (R&D Systems, Wiesbaden-Nordenstadt) at 25 ng/ml carried on 0.1% human albumin (CSL-Behring, Hattersheim-am-Main). On day 6 of culture, cells were stimulated for a further 18-24 hours with 25 ng/mL rhIFN-γ (Chemicon, Billerica, MA). IFN-γ-stimulated macrophages (IFN-γ-Mφ) were generated by cultivating CD14+ monocytes under identical conditions to Mregs except that human serum was replaced with 10% heat-inactivated fetal calf serum (FCS) (Biochrom, Berlin). Macrophages (Mφ) in other defined states of polarisation were generated from positively-isolated CD14+ monocytes according to protocols adapted from the literature [12]-[15]. Briefly, monocytes were cultured for 7-days at 10⁵ cells/cm² in Cell+ plastic ware (Sarstedt) in RPMI-based medium with 100U/ml penicillin, 100 µg/ml streptomycin and 2mM GlutaMAXTM, supplemented as follows: Resting Mφ - 20% FCS plus 100 ng/ml M-CSF for 7 days; lipopolysaccharide (LPS)-activated Mφ - 20% FCS plus 100 ng/ml M-CSF with addition of 100 ng/ml LPS (Enzo Life Sciences) and 20 ng/ml IFN-γ on day 6; IL-4-stimulated Mφ - 20% FCS plus 100 ng/ml M-CSF with addition of 20 ng/ml IL-4 (R&D Systems) on day 6; Ig-stimulated Mφ - 10% FCS plus 100 ng/ml M-CSF, cells grown in plastic ware precoated with human IVIg (PrivigenTM, CSL Behring) and with addition of 100 ng/ml LPS on day 6; Glucocorticoid (GC)-stimulated Mφ - 20% FCS plus 100 ng/ml M-CSF with addition of 10-7 M dexamethasone (Sigma-Aldrich) on day 6.

A series of monoclonal antibodies (mAb) were generated by vaccinating mice with human Mreg lysates. Screening these mAb by immunocytochemistry identified a mAb clone (ASOT1) that reacted strongly with Mregs, but not other monocyte-derived macrophages (Mφ), including resting Mφ, LPS + IFNγ-stimulated Mφ, IL-4-stimulated Mφ and immunoglobulin (Ig)-stimulated Mφ (see Figure 3A). By immunoprecipitating and sequencing its antigen, the ASOT1 mAb was shown to recognise DHRS9, a little-studied retinol dehydrogenase of the SDR family (see Figure 3B). Quantitative PCR confirmed that DHRS9 mRNA expression is restricted to Mregs (Fig. 3C). A rabbit polyclonal antibody generated against an N-terminal epitope of DHRS9 reacted against a protein of about 35 kD immunoprecipitated by ASOT1 (see Figure 3D). As a commercially-available monoclonal antibody (clone 3C6) which recognises DHRS9 also reacted with the same protein detected by the rabbit antibody, it can be confidently concluded that both ASOT1 and the rabbit polyclonal antibody recognise DHRS9. Using this rabbit pAb, DHRS9 protein expression was shown to be unique to Mregs (Fig. 3E).

Whole genome expression profiling was used to gain a global view of the phenotypic proximity between Mregs and macrophages in other activation states. Microarray analyses were performed with a panel of nine comparator macrophage types, which were prepared from three separate donors in parallel. Genes which were more than 20-fold differentially expressed between any two samples were selected and clustered hierarchically, revealing that the Mreg samples were most similar to IFN-γ-untreated Mregs and LPS-stimulated Mregs than any other macrophage sample. This clustering pattern remained stable when all significantly differentially-expressed probes were used for the analysis. The Mreg samples were more similar to Ig-stimulated M2b macrophages than to other macrophage types, underscoring the importance of stimulation with Ig in the development of the Mreg phenotype according to aspect 1, step (c). Resting macrophages and IFN-γ-stimulated macrophages clustered with M2a and M2c macrophages. Classically-activated M1 macrophages were more dissimilar to the comparator macrophages types than the comparator macrophages were to each other. From these observations, it may be concluded that Mregs are in a unique state of activation and are relatively refractory to repolarisation towards the M1 phenotype by stimulation with LPS.

The microarray array results were consistent with the flow cytometry findings in so far as CD163, IL-10 and CD14 were found within the list of down-regulated genes that discriminated Mregs from all other comparator macrophages. Within the gene set uniquely up-regulated by Mregs, CD258 (TNFSF14, LIGHT) and CD85H (ILT1, LILRA2) were identified as useful markers for Mreg identity. Expression of CD258 and CD85b by Mregs, but not by IFN-γ-stimulated macrophages, was confirmed by flow cytometry (Fig. 7).

The constellation of CD45⁺ CD11b⁺ CD11c⁺ CD14^{-/low} CD209^{-/low} CD16^{-/low} CD80^{-/low} CD86⁺ CD10^{+/-} CD103^{+/-} CD38^{-/low} CD85h⁺ CD258⁺ Syndecan-3^{-/low} Clec-9a⁺ DHRS9⁺ and Arg-1⁺ and IDO⁺ is a rigorous and stable definition of the phenotype of Mregs.

### Example 4: Generation of activated peripherally-induced human Tregs by allogeneic Mreg-bc treatment in NOD/SCID/IL2rγ^{null} mice

The Mreg-bc cells of the invention were prepared as described in Example 1. Immunodeficient NOD/SCID/IL2rγ^{null} (NSG) mice were reconstituted with human T cells. These mice were either treated with Mreg-bc cells of the invention or not (see Figure 8A). Human T cells were recovered from the spleens of recipient mice at 5 days after Mreg-bc cell treatment. The T cell population in Mreg-treated mice was enriched for FoxP3⁺ Tregs and TIGIT⁺ FoxP3⁺ Tregs compared to recipients that were not treated with Mregs-bc (see Figure 8B). Serum levels of human IL-10 were significantly higher in NSG mice that were treated with Mreg-bc cells compared to untreated animals (see Figure 8C). This example demonstrates that human Mregs-bc can directly interact with allogeneic human T cells *in vivo* to induce Treg development.

### Example 5: Treatment of a kidney transplant recipient with Mreg-bc cells prior to surgery

The Mreg-bc cells of the invention were prepared as described in Example 1. The Mreg-bc cells were administered to a 43 year old prospective living-donor kidney transplant recipient with end-stage renal failure owing to polycystic kidney disease. The Mreg-bc cells were produced from monocytes collected from the recipient's healthy 62 year old father, who subsequently donated a kidney to his son. Donor and recipient had single mismatches at the HLA-A, -B and -DR loci.

A total of 4.75 × 10⁸ viable Mregs-bc were administered by slow central venous infusion. No adverse reactions were encountered. Specifically, there was no evidence of pulmonary vascular obstruction, right heart strain, transfusion reactions, hypersensitivity reactions or biochemical disturbances. Treatment with Mreg-bc cells did not cause the recipient to produce anti-donor HLA antibodies.

The recipient is now more than 15 months post-transplant with stable allograft function. The recipient is currently maintained with a reduced-dose immunosuppressive regimen comprising tacrolimus and MMF. This case illustrates the feasibility of administering Mreg-bc cells to a preoperative kidney transplant recipient.

### Example 6: Production of angiogenic factors by Mreg-bc cells

It was tested whether Mreg-bc cells produce angiogenic factor VEGF-A upon stimulation with monophosphoryl lipid A (MPLA). The set-up of this experiment is depicted in Figure 9.

In the first test condition, Mreg-bc were grown as described in Example 1 until day 7, including standard 25 ng/ml IFN-γ stimulation of day 6. On day 7, the Mreg-bc cells were harvested and replated in 1 ml of RPMI-1640 + 1% HABS + Pen-Strep + 2 mM GlutaMAX at 0.5 × 10⁶ cells per well in a 24-well plate. These replated Mreg-bc cells were then either stimulated with 1 µg/ml LPS or not. In parallel, Mreg-bc cells were harvested and investigated by flow cytometry for CD14, CD10, CD16, CD38, CD80, CD86, CD85h, CD103, CD258, CD209 and Syndecan-3.

In the second condition, the Mreg-bc cells were additionally stimulated with 100 ng/ml MPLA on day 6 of culture at the same time IFN-γ was added. On day 7, harvested Mreg-bc cells from condition 2 were re-plated and stimulated as condition 1. In addition, Mreg-bc cells from condition 2 were analysed by flow cytometry for the markers CD14, CD10, CD16, CD38, CD80, CD86, CD85h, CD103, CD258, CD209 and Syndecan-3.

In the third condition, Mreg-bc cells were stimulated with 25 ng/ml IFN-γ as usual on day 6. On day 7, the cells were further stimulated with 100 ng/ml MPLA for another 24 hours. The cells were then harvested on day 8 for analysis as conditions 1 and 2.

Secretion of VEGF-A by the cells from all three conditions was measured by ELISA. The phenotype of cells from all three conditions was compared by flow cytometry to assess the stability of the Mreg-bc-defining cell-surface phenotype under the test conditions.

Result: The result of the VEGF-A determination is depicted in Figure 9. It was found that treatment with 100 ng/ml MPLA on day 6 or day 7 augments the LPS-induced expression of VEGF. Within 24 hours, treatment with 100 ng/ml MPLA does not dramatically alter the Mreg-bc phenotype: only minor up-regulation of CD80 expression was observed. These examples indicate that MPLA treatment during Mreg-bc culture could be a useful way of enhancing VEGF-A production by Mreg-bc cells before application to the patient.

### Example 7: Production of angiogenic factors by Mreg-bc cells

It was tested whether Mreg-bc cells produce angiogenesis-related factors. For this purpose, more than 30 × 10⁶ CD14+ monocytes/donor were isolated from 5 fresh donors. Medium (RPMI-1640 + 10% HABS + 2 mM GlutaMax + PS + 25 ng/ml recombinant human M-CSF) was prepared for 5 × 500 ml bags. One 500 ml bag per donor was filled with 30 × 10⁶ monocytes/bag. On day 6, all bags were stimulated with IFN-γ. On day 7, Mregs were harvested and counted.

Subsequently, Mreg medium (RPMI-1640 + 10% HABS + 2 mM GlutaMax + PS + 25 ng/ml recombinant human M-CSF) was prepared for a re-culture. Exactly 15 ml medium were added to each of 8 × 50 ml tubes. NaCl solution or 10 ng/ml LPS (Enzo) was added as follows and vortexed:

| Condition | Stimulation | + NaCl (mM) |
|---|---|---|
| 1 | NaCl | 0 |
| 2 | NaCl | 5 |
| 3 | NaCl | 10 |
| 4 | NaCl | 20 |
| 5 | NaCl | 40 |
| 6 | NaCl | 80 |
| 7 | NaCl | 160 |
| 8 | LPS | 0 |

Mregs were plated at 1 × 10⁶ cells/well in 24-well plates. 1 well per condition and donor:

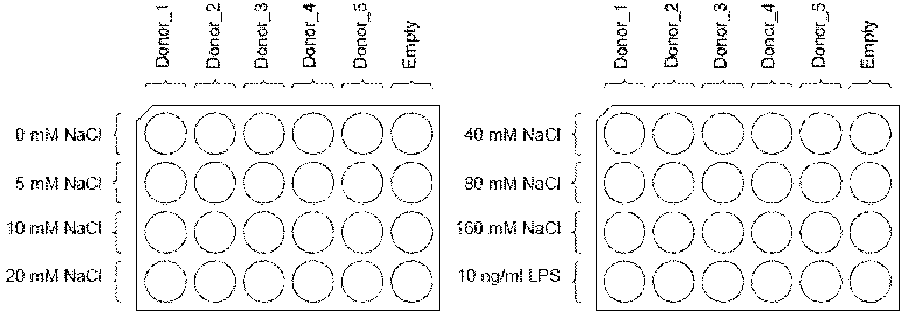

The cultures were incubated for exactly 48 hours. Supernatants were harvested and cleared. 2 aliquots of ≥ 500 µl were prepared. The samples were stored at -80°C until analysis by ELISA for VEGF-A, VEGF-C, VEGF-D and TNF-α.

Result: The result of the ELISA is shown in Figure 10. As can be seen in Figure 10A, Mregs in combination with LPS induced VEGF-A, VEGF-C, and TNF-α, but not VEGF-D. Under hyper-tionic conditions, Mregs were induced to express VEGF-C but not VEGF-A, VEGF-D or TNF-α (Figure 10B).

### LITERATURE

[1] Geissler EK, Hutchinson JA. Cell therapy as a strategy to minimize maintenance immunosuppression in solid organ transplant recipients. Curr Opin Organ Transplant 2013; 18: 408-15.
[2] Tang Q, Bluestone JA, Kang SM. CD4(+) Foxp3(+) regulatory T cell therapy in transplantation. J Mol Cell Biol 2012; 4: 11-21.
[3] Moreau A, Varey E, Bouchet-Delbos L, et al. Cell therapy using tolerogenic dendritic cells in transplantation. Transplant Res 2012; 1: 13.
[4] Broichhausen C, Riquelme P, Geissler EK, et al. Regulatory macrophages as therapeutic targets and therapeutic agent in solid organ transplantation. Curr Opin Organ Transplant 2012; 17: 332-42.
[5] Hutchinson JA, Riquelme P, Sawitzki B, et al. Cutting edge: immunological consequences and trafficking of human regulatory macrophages administered to renal transplant recipients. J Immunol 2011; 187: 2072-8.
[6] Hutchinson JA, Riquelme P, Brem-Exner BG, et al. Tranplant acceptance-inducing cells as an immune-conditioning therapy in renal transplantation. Transpl Int 2008; 21: 728-41.
[7] Hutchinson JA, Brem-Exner BG, Riquelme P, et al. A cell-based approach to the minimization of immunosuppression in renal transplantation. Transpl Int 2008; 21: 742-54.
[8] Hutchinson JA, Roelen D, Riquelme P, et al. Preoperative treatment of a pre-sensitized kidney transplant recipient with donor-derived transplant acceptance-inducing cells. Transpl Int 2008; 21: 808-13.
[9] Hutchinson JA, Govert F, Riquelme P, et al. Administration of donor-derived transplant acceptance-inducing cells to the recipients of renal transplants from deceased donors is technically feasible. Clin Transplant 2009; 23: 140-5.
[10] Hutchinson JA, Riquelme P, Geissler EK, and Fändrich F. Human regulatory macrophages. Methods Mol. Biol. 2011; 677: 181-192.
[11] Riquelme P, Tomiuk S, Kammler A, Fändrich F, Schlitt HJ, Geissler EK, Hutchinson JA. Mol Ther. 2013; 21(2):409-22.
[12] Martinez FO, Gordon S, Locati M, Mantovani A. J Immunol 2006;177: 7303-7311.
[13] Munn DH, Shafizadeh E, Attwood JT, Bondarev I, Pashine A, Mellor AL. J Exp Med 1999;189: 1363-1372.
[14] Sironi M, Martinez FO, D'Ambrosio D et al. J Leukoc Biol 2006;80: 342-349.
[15] Kzhyshkowska J, Workman G, Cardo-Vila M et al. J Immunol 2006;176: 5825-5832.
[16] Cao Y, Linden P, Farnebo J, Cao R, Eriksson A, Kumar V, Qi JH, Claesson-Welsh L, Alitalos K, Proc. Natl. Acad. Sci. USA 1998; 95: 14389-14394.

## Claims

1. A process for preparing an immunoregulatory macrophage cell, said process comprising:
(a) isolating CD14 positive monocytes from a blood sample of a subject;
(b) culturing the monocytes in a gas-permeable plastic bag in a culture medium containing (i) M-CSF and (ii) a CD16 ligand, wherein the concentration of M-CSF is in the range of 5-100 ng/ml;
(c) contacting the cells with IFN-γ wherein the concentration of IFN-γ is in the range of 5-100 ng/ml; and
(d) obtaining the immunoregulatory macrophage cell from the culture medium,
wherein the monocytes in step (b) are cultured for at least 5 days prior to IFN-γ stimulation.

2. Process of claim 1, wherein the culture medium in step (b) comprises human blood serum, such as human AB serum.

3. Process of claim 1 or 2, wherein the concentration of M-CSF in step (b) is in the range of 20-25 ng/ml.

4. Process of any of claims 1-3, wherein the monocytes in step (b) are cultured for at least 6, or for at least 7 days prior to IFN-γ stimulation.

5. Process of any of claims 1-4, wherein the gas-permeable plastic bag is made of polyolefine.

6. Process of any of claims 1-5, wherein the concentration of IFN-γ in step (c) is in the range of 20-25 ng/ml.

7. Immunoregulatory macrophage cell, wherein said cell
(a) expresses at least one of the markers CD103, CD10 and Clec-9a; and
(b) wherein the expression of the marker CD38 is downregulated by more than 70% relative to the expression of said marker by monocytes.

8. Immunoregulatory macrophage cell of claim 7, wherein said cell does not express one or more of the following markers: CD38, CD209 and Syndecan-3.

9. Immunoregulatory macrophage cell of any of claims 7 or 8, wherein said cell further expresses at least one of the markers CD85h and CD258.

10. Pharmaceutical composition comprising the immunoregulatory macrophage cell of any of claims 7-9.

11. Immunoregulatory macrophage cell of any of claims 7-9 or pharmaceutical composition of claim 10 for use in a method of
(a) suppressing transplant rejection and/or prolonging transplant survival in a subject receiving a transplant, preferably an allogeneic transplant;
(b) treating or preventing an autoimmune disease, an inflammatory disease, or a hypersensitivity reaction; or
(c) promoting tissue-repair processes by participating in tissue remodelling, tissue regeneration, angiogenesis, vasculogenesis, or prevention/limitation of fibrosis.

12. Immunoregulatory macrophage cell or pharmaceutical composition for use in a method of claim 11, wherein said autoimmune disease is selected from the group consisting of systemic lupus erythematosus (SLE), scleroderma, Sjögren's syndrome, polymyositis, dermatomyositis, and other systemic autoimmune conditions; rheumatoid arthritis (RA), juvenile rheumatoid arthritis, and other inflammatory arthritides; ulcerative colitis, Crohn's disease, and other inflammatory bowel diseases; autoimmune hepatitis, primary biliary cirrhosis, and other autoimmune liver diseases; cutaneous small-vessel vasculitis, granulomatosis with polyangiitis, eosinophilic granulomatosis with polyangiitis, Behçet's disease, thromboangiitis obliterans, Kawasaki disease, and other large-, medium- or small-vessel vasculitides of autoimmune aetiology; Multiple sclerosis (MS) and neuroimmunological disorders; Type I diabetes, autoimmune thyroid dysfunction, autoimmune pituitary dysfunction, and other autoimmune endocrinological disorders; haemolytic anaemia, thrombocytopaenic purpura and other autoimmune disorders of the blood and bone marrow; psoriasis, pemphigus vulgaris, pemphigoid and other autoimmune dermatological conditions.

13. Immunoregulatory macrophage cell or pharmaceutical composition for use in a method of claim 11, wherein said inflammatory disease is selected from the group consisting of arterial occlusive diseases, such as peripheral artery occlusive disease (pAOD), critical limb ischaemia, arteriosclerosis, cerebral infarction, myocardial infarction, renal infarction, intestinal infarction, angina pectoris, and other conditions caused by arterial occlusion or constriction; microvascular angina, also known as cardiac syndrome X; inflammation associated systemic with metabolic disorders, including Type II diabetes and obesity-related metabolic syndrome; dermatological diseases, including eczema.

14. Immunoregulatory macrophage cell or pharmaceutical composition for use in a method of claim 11, wherein said hypersensitivity reaction is selected from the group consisting of asthma, eczema, allergic rhinitis, angioedema, drug hypersensitivity and mastocytosis.

15. A process for preparing an immunoregulatory T cell, said process comprising:
(a) obtaining T cells of a subject;
(b) co-culturing the T cells with an immunoregulatory macrophage cell of any of claims 7-9;
(c) obtaining the immunoregulatory T cell from the culture medium.

## Patentansprüche

1. Verfahren zur Herstellung einer immunregulatorischen Makrophagen-Zelle, bei dem man
(a) CD14-positive Monozyten aus einer Blutprobe eines Subjekts isoliert;
(b) die Monozyten in einem gaspermeablen Kunststoffbeutel in einem Kulturmedium anzüchtet, das (i) M-CSF und (ii) einen CD16-Liganden enthält, wobei die Konzentration von M-CSF im Bereich von 5-100 ng/ml liegt;
(c) die Zellen mit IFN-γ in Kontakt bringt, wobei die Konzentration von IFN-γ im Bereich von 5-100 ng/ml liegt; und
(d) die immunregulatorischen Makrophagen-Zellen aus dem Kulturmedium gewinnt,
wobei die Monozyten in Schritt (b) vor der IFN-γ-Stimulation für mindestens 5 Tage kultiviert werden.

2. Verfahren nach Anspruch 1, wobei das Kulturmedium in Schritt (b) humanes Blutserum, wie beispielsweise humanes AB-Serum, umfasst.

3. Verfahren nach Anspruch 1 oder 2, wobei die Konzentration von M-CSF in Schritt (b) im Bereich von 20-25 ng/ml liegt.

4. Verfahren nach einem der Ansprüche 1-3, wobei die Monozyten in Schritt (b) vor der IFN-γ-Stimulation für mindestens 6 oder mindestens 7 Tage kultiviert werden.

5. Verfahren nach einem der Ansprüche 1-4, wobei der gaspermeable Kunststoffbeutel aus Polyolefin besteht.

6. Verfahren nach einem der Ansprüche 1-5, wobei die Konzentration von IFN-γ in Schritt (c) im Bereich von 20-25 ng/ml liegt.

7. Immunregulatorische Makrophagen-Zelle, wobei die Zelle
(a) mindestens einen der Marker CD103, CD10 und Clec-9a exprimiert; und
(b) wobei die Expression des Markers CD38 um mehr als 70% im Vergleich zur Expression des Markers durch Monozyten herunterreguliert ist.

8. Immunregulatorische Makrophagen-Zelle nach Anspruch 7, wobei die Zelle einen oder mehrere der folgenden Marker nicht exprimiert: CD38, CD209 und Syndecan-3.

9. Immunregulatorische Makrophagen-Zelle nach einem der Ansprüche 7 oder 8, wobei die Zelle ferner mindestens einen der Marker CD85h und CD258 exprimiert.

10. Pharmazeutische Zusammensetzung, die die immunregulatorische Makrophagen-Zelle nach einem der Ansprüche 7-9 enthält.

11. Immunregulatorische Makrophagen-Zelle nach einem der Ansprüche 7-9 oder pharmazeutische Zusammensetzung nach Anspruch 10 zur Verwendung in einem Verfahren zur
(a) Unterdrückung der Transplantat-Abstoßung und/oder Verlängerung des Transplantatüberlebens in einem Subjekt, das ein Transplantat erhält, vorzugsweise ein allogenes Transplantat;
(b) Behandlung oder Vorbeugung einer Autoimmunerkrankung, einer entzündlichen Erkrankung oder einer Überempfindlichkeitsreaktion; oder
(c) Förderung von Gewebereparaturprozessen durch Beteiligung an dem Gewebeumbau, Geweberegeneration, Angiogenese, Vaskulogenese oder Vermeidung/Begrenzung von Fibrose.

12. Immunregulatorische Makrophagen-Zelle oder pharmazeutische Zusammensetzung zur Verwendung in einem Verfahren nach Anspruch 11, wobei die Autoimmunerkrankung ausgewählt ist aus der Gruppe bestehend aus systemischem Lupus erythematodes (SLE), Sklerodermie, Sjögren-Syndrom, Polymyositis, Dermatomyositis und anderen systemischen Autoimmunerkrankungen; rheumatoider Arthritis (RA), juveniler rheumatoider Arthritis und anderen entzündlichen Arthritiden; Colitis ulcerosa, Morbus Crohn und anderen entzündlichen Darmerkrankungen; autoimmuner Hepatitis, primärer biliärer Zirrhose und anderen autoimmunen Lebererkrankungen; kutaner Vaskulitis der kleinen Gefäße, Granulomatose mit Polyangiitis, eosinophiler Granulomatose mit Polyangiitis, Morbus Behçet, Thromboangiitis obliterans, Kawasaki-Krankheit und anderen Vaskulitiden der großen, mittleren oder kleinen Gefäße mit autoimmuner Ätiologie; Multiple Sklerose (MS) und neuroimmunologischen Erkrankungen; Typ-I-Diabetes, autoimmuner Schilddrüsenfehlfunktion, autoimmuner Hypophysenfehlfunktion und anderen autoimmunen endokrinologischen Erkrankungen; hämolytische Anämie, thrombozytopenischer Purpura und anderen autoimmunen Erkrankungen von Blut und Knochenmark; Psoriasis, Pemphigus vulgaris, Pemphigoid und anderen autoimmunen dermatologische Erkrankungen.

13. Immunregulatorische Makrophagen-Zelle oder pharmazeutische Zusammensetzung zur Verwendung in einem Verfahren nach Anspruch 11, wobei die entzündliche Erkrankung ausgewählt ist aus der Gruppe bestehend aus arteriellen Verschlusskrankheiten, wie peripherer arterieller Verschlusskrankheit (PAVK), kritischer Extremitätenischämie, Arteriosklerose, zerebralem Infarkt, Myokardinfarkt, Niereninfarkt, Darminfarkt, Angina pectoris und anderen Zuständen, die durch arteriellen Verschluss oder arterielle Verengung verursacht werden; mikrovaskulärer Angina pectoris, auch bekannt als kardiales Syndrom X; Entzündungen in Verbindung mit Stoffwechselstörungen, einschließlich Typ-II-Diabetes und durch Fettleibigkeit bedingtes metabolisches Syndrom; dermatologischen Erkrankungen, einschließlich Ekzem.

14. Immunregulatorische Makrophagen-Zelle oder pharmazeutische Zusammensetzung zur Verwendung in einem Verfahren nach Anspruch 11, wobei die Überempfindlichkeitsreaktion ausgewählt ist aus der Gruppe bestehend aus Asthma, Ekzem, allergischer Rhinitis, Angioödem, Arzneimittelüberempfindlichkeit und Mastozytose.

15. Verfahren zur Herstellung einer immunregulatorischen T-Zelle, bei dem man
(a) T-Zellen eines Subjekts gewinnt;
b) die T-Zellen mit einer immunregulatorischen Makrophagen-Zelle nach einem der Ansprüche 7-9 co-kultiviert;
c) die immunregulatorische T-Zellen aus dem Kulturmedium gewinnt.

## Revendications

1. Procédé de préparation d'un macrophage immuno-régulateur, lequel procédé comporte les étapes suivantes :
a) isoler des monocytes CD14-positifs, à partir d'un échantillon de sang d'un sujet,
b) cultiver ces monocytes, dans un sac en plastique perméable aux gaz, dans un milieu de culture qui contient (i) du M-CSF et (ii) un ligand de CD16 et dans lequel la concentration du M-CSF se situe dans l'intervalle allant de 5 à 100 ng/mL,
c) mettre ces cellules en contact avec de l'IFN-γ, étant entendu que la concentration de l'IFN-γ se situe dans l'intervalle allant de 5 à 100 ng/mL,
d) et récupérer les macrophages immuno-régulateurs à partir du milieu de culture,
étant entendu que dans l'étape (b), les monocytes sont cultivés durant au moins 5 jours avant leur stimulation par l'IFN-γ.

2. Procédé conforme à la revendication 1, dans lequel le milieu de culture de l'étape (b) comprend du sérum sanguin humain, comme du sérum AB humain.

3. Procédé conforme à la revendication 1 ou 2, dans lequel, dans l'étape (b), la concentration du M-CSF se situe dans l'intervalle allant de 20 à 25 ng/mL.

4. Procédé conforme à l'une des revendications 1 à 3, dans lequel, dans l'étape (b), les monocytes sont cultivés durant au moins 6 jours ou au moins 7 jours avant leur stimulation par l'IFN-γ.

5. Procédé conforme à l'une des revendications 1 à 4, dans lequel le sac en plastique perméable aux gaz est en polyoléfine.

6. Procédé conforme à l'une des revendications 1 à 5, dans lequel, dans l'étape (c), la concentration de l'IFN-γ se situe dans l'intervalle allant de 20 à 25 ng/mL.

7. Cellule macrophage immuno-régulateur, laquelle cellule
a) exprime au moins l'un des marqueurs CD103, CD10 et Clec-9a,
b) et dans laquelle l'expression du marqueur CD38 est régulée en baisse de plus de 70 % par rapport à l'expression de ce marqueur chez les monocytes.

8. Cellule macrophage immuno-régulateur, conforme à la revendication 7, laquelle cellule n'exprime pas l'un ou plusieurs des marqueurs suivants : CD38, CD209 et Syndecan-3.

9. Cellule macrophage immuno-régulateur, conforme à l'une des revendications 7 et 8, laquelle cellule exprime en outre au moins l'un des marqueurs CD85h et CD258.

10. Composition pharmaceutique comprenant un macrophage immuno-régulateur, conforme à l'une des revendications 7 à 9.

11. Macrophage immuno-régulateur, conforme à l'une des revendications 7 à 9, ou composition pharmaceutique conforme à la revendication 10, pour utilisation dans un procédé visant
a) à supprimer le rejet d'un greffon et/ou à prolonger la survie d'un greffon, chez un sujet receveur d'un greffon, de préférence un greffon allogène,
b) à traiter ou à prévenir une maladie auto-immune, une maladie inflammatoire ou une réaction d'hypersensibilité,
c) ou à promouvoir un processus de réparation de tissu par participation à un remodelage de tissu, à une régénération de tissu, à une angiogenèse, à une vasculogenèse, ou à la prévention/limitation d'une fibrose.

12. Macrophage immuno-régulateur ou composition pharmaceutique pour utilisation dans un procédé, conforme à la revendication 11, étant entendu que ladite maladie auto-immune est choisie dans l'ensemble constitué par les suivantes : lupus érythémateux systémique (SLE), sclérodermie, syndrome de Sjögren, polymyosite, dermatomyosite, et autres maladies auto-immunes systémiques ; polyarthrite rhumatoïde (RA), polyarthrite rhumatoïde juvénile, et autres arthrites inflammatoires ; colite ulcéreuse, maladie de Crohn, et autres maladies intestinales inflammatoires ; hépatite auto-immune, cirrhose biliaire primitive, et autres maladies hépatiques auto-immunes ; vascularite des petits vaisseaux cutanés, granulomatose avec polyangéite, granulomatose éosinophile avec polyangéite, maladie de Behçet, thromboangéite oblitérante, maladie de Kawasaki, et autres vascularites d'étiologie auto-immune, affectant les grands, moyens ou petits vaisseaux ; sclérose en plaques (SEP) et troubles neuro-immunologiques ; diabète de type I, dysfonctionnement auto-immun de la thyroïde, dysfonctionnement auto-immun de l'hypophyse, et autres troubles endocriniens auto-immuns ; anémie hémolytique, purpura thrombocytopénique et autres troubles auto-immuns du sang et de la moelle osseuse ; psoriasis, pemphigus vulgaire, pemphigoïde et autres maladies dermatologiques auto-immunes.

13. Macrophage immuno-régulateur ou composition pharmaceutique pour utilisation dans un procédé, conforme à la revendication 11, étant entendu que ladite maladie inflammatoire est choisie dans l'ensemble constitué par les suivantes : maladies occlusives artérielles, telles que maladie occlusive des artères périphériques (pAOD), ischémie aiguë d'un membre, artériosclérose, infarctus cérébral, infarctus du myocarde, infarctus rénal, infarctus intestinal, angine de poitrine, et autres troubles causés par l'occlusion ou le rétrécissement d'une artère ; angor microvasculaire, également appelé syndrome cardiaque X ; inflammation systémique associée à des troubles du métabolisme, y compris diabète de type II et syndrome métabolique lié à l'obésité ; maladies dermatologiques, y compris eczéma.

14. Macrophage immuno-régulateur ou composition pharmaceutique pour utilisation dans un procédé, conforme à la revendication 11, étant entendu que ladite réaction d'hypersensibilité est choisie dans l'ensemble constitué par les suivantes : asthme, eczéma, rhinite allergique, œdème de Quincke, hypersensibilité médicamenteuse et mastocytose.

15. Procédé de préparation d'un lymphocyte T immuno-régulateur, lequel procédé comporte les étapes suivantes :
a) prélever des lymphocytes T chez un sujet,
b) co-cultiver ces lymphocytes T avec des macrophages immuno-régulateurs, conformes à l'une des revendications 7 à 9,
c) et récupérer les lymphocytes T immuno-régulateurs à partir du milieu de culture.
